# EUROPEAN PATENT APPLICATION

(11) **EP 4 328 922 A1**
(43) Date of publication of application: **28.02.2024**
(21) Application number: 21937955.9
(22) Date of filing: 17.08.2021
(51) Int. Cl.: G16H 10/20

(54) **BLOOD SUGAR CONSTITUTION ASSESSMENT DEVICE, BLOOD SUGAR CONSTITUTION ASSESSMENT METHOD, AND RECORDING MEDIUM**

(30) Priority: 20.04.2021 JP 2021071175
(71) Applicant: Suntory Holdings Limited, Kita-ku, Osaka-shi Osaka 530-8203 (JP)
(72) Inventor: UCHIDA Tomoki, Soraku-gun, Kyoto 619-0284 (JP); MURAYAMA Norihito, Soraku-gun, Kyoto 619-0284 (JP); NONAKA Yuji, Tokyo 135-8631 (JP); KANAMORI Takeshi, Soraku-gun, Kyoto 619-0284 (JP)
(74) Representative: Vossius & Partner Patentanwälte Rechtsanwälte mbB
(86) International application number: PCT/JP2021/030047
(87) International publication number: WO 2022/224465

(57) **Abstract**

Conventionally, it has not been possible to easily know the user's blood sugar constitution type. It is possible to easily know the user's blood sugar constitution type using a blood sugar constitution determination device including: a storage unit that stores one or more pieces of question information that are used to judge a blood sugar constitution type and include a question regarding lifestyle; an output unit that outputs the one or more pieces of question information; an acceptance unit that accepts, from a user, answer information corresponding to the one or more pieces of question information; a processing unit that determines a blood sugar constitution type of the user, using the one or more pieces of answer information accepted by the acceptance unit; and an information output unit that outputs output information regarding the blood sugar constitution type.

## Description

### Technical Field

The present invention relates to a blood sugar constitution determination device that determines a blood sugar constitution type.

### Background Art

Conventionally, health condition has been predicted from the results of a blood test or the like during a health checkup. In addition, pre-diabetes and diabetes risk have been predicted from general risk factors. More specifically, conventionally, for example, a user's health condition with respect to blood sugar has been observed using a method called a sugar tolerance test. Specifically, for example, after the user ingests 75 g of sugar, a blood sample is taken every 30 minutes until 120 minutes have elapsed, the blood is tested, the blood sugar level is measured to acquire changes in the user's blood sugar level over time, the changes over time are referenced, and the user's health condition determined by a doctor with respect to blood sugar is observed.

Furthermore, a blood sugar level information management device capable of presenting trigger information to prompt a patient to continuously perform an action to improve their own blood sugar level has been conventionally available (see Patent Document 1). Such a device is a blood sugar level management device in which medical information acquisition devices used by patients are registered, configured to: generate biometric information change information including blood sugar level change information that is information regarding changes in blood sugar level within a predetermined period, based on biometric information including blood sugar level information acquired from each of the medical information acquisition devices; acquire answer information based on lifestyle question information, which is questions regarding the patient's lifestyle within the predetermined period; and generate improvement degree display information graphically showing the degree of improvement in lifestyle, based on the answer information; at least the biometric information change information, the lifestyle question information, answer information corresponding thereto, and improvement degree display information are pieces of data that can be displayed on one sheet, the lifestyle question information includes only actions that contribute to improvement of blood sugar level, and the improvement degree display information indicates a degree of execution of an action that contributes to improvement of blood sugar level.

### Citation List

### Patent Document

Patent Document 1: JP 2016-48531A

### Summary of Invention

### Technical Problem

However, with the conventional method or the conventional technology, it is not possible to easily know the user's blood sugar constitution type. In addition, the conventional technology cannot provide user with appropriate advice according to the type of the user's blood sugar constitution type.

### Solution to Problem

A blood sugar constitution determination device according to a first aspect of the present invention is a blood sugar constitution determination device including: a storage unit that stores one or more pieces of question information that are used to judge a blood sugar constitution type and include a question regarding lifestyle; an output unit that outputs the one or more pieces of question information; an acceptance unit that accepts, from a user, answer information corresponding to the one or more pieces of question information; a processing unit that determines a blood sugar constitution type of the user, using the one or more pieces of answer information accepted by the acceptance unit; and an information output unit that outputs output information regarding the blood sugar constitution type.

With such a configuration, it is possible to easily know the user's blood sugar constitution type.

A blood sugar constitution determination device according to a second aspect of the present invention is the blood sugar constitution determination device according to the first aspect of the invention, wherein the storage unit includes: a first question storage unit that stores one or more first questions that are used to judge presence or absence of a risk related to blood sugar and include a question regarding lifestyle; and a second question storage unit that stores one or more second questions that are used to judge a blood sugar constitution type and include a question regarding lifestyle, the one or more pieces of question information are the first questions or the second questions, the output unit includes: a first question output unit that outputs the one or more first questions; and a second question output unit that outputs the one or more second questions when the first judgment unit judges that a risk is present, the acceptance unit includes: a first answer acceptance unit that accepts first answers to the one or more first questions from the user; and a second answer acceptance unit that accepts second answers to the one or more second questions from the user, and the processing unit includes: a first judgment unit that judges presence or absence of a risk related to a blood sugar of the user, using the one or more first answers accepted by the first answer acceptance unit; and a second judgment unit that determines a blood sugar constitution type of the user, using the one or more second answers accepted by the second answers acceptance unit.

With such a configuration, it is possible to easily know the user's blood sugar constitution type. Furthermore, more specifically, by providing questions to users in two stages, it is possible for users with no blood sugar risk to determine that there is no risk, using answers to a small number of questions, which reduces the burden on the users.

A blood sugar constitution determination device according to a third aspect of the present invention is the blood sugar constitution determination device according to the second aspect of the invention, wherein the second judgment unit additionally uses the one or more first answers accepted by the first answer acceptance unit to determine the blood sugar constitution type of the user.

With such a configuration, it is possible to accurately determine the user's blood sugar constitution type. Furthermore, more specifically, the blood sugar constitution type of each user is determined in the second step using the first answers as well, and therefore a user who has a risk related to blood sugar can determine the blood sugar constitution type, using answers to a small number of questions, which reduces the burden on the users.

A blood sugar constitution determination device according to a fourth aspect of the present invention is the blood sugar constitution determination device according to the first aspect of the invention, further including: a learner storage unit that stores a learner acquired by performing machine learning processing on two or more pieces of training data that each contain one or more pieces of answer information and a blood sugar constitution type, wherein the processing unit acquires a blood sugar constitution type by performing machine learning prediction processing, using the one or more pieces of answer information accepted by the acceptance unit and the learner.

With such a configuration, it is possible to accurately determine the user's blood sugar constitution type.

A blood sugar constitution determination device according to a fifth aspect of the present invention is the blood sugar constitution determination device according to the second or third aspect of the invention, further including: a learner storage unit that stores a learner acquired by performing machine learning processing on two or more pieces of training data that each contain one or more second answers and a blood sugar constitution type, wherein the second judgment unit acquires a blood sugar constitution type by performing machine learning prediction processing, using the one or more second answers accepted by the second acceptance unit and the learner.

With such a configuration, it is possible to accurately determine the user's blood sugar constitution type.

A blood sugar constitution determination device according to a sixth aspect of the present invention is the blood sugar constitution determination device according to any one of the first to fifth aspects of the invention, further including: an advice storage unit that stores one or more pieces of advice information in association with one or more type identifiers identifying the blood sugar constitution type, wherein the information output unit outputs output information that contains one or more pieces of advice information associated with a type identifier identifying the type determined by the second judgment unit.

With such a configuration, it is possible to present an advance corresponding to the user's blood sugar constitution type.

A blood sugar constitution determination device according to a seventh aspect of the present invention is the blood sugar constitution determination device according to the sixth aspect of the invention, further including: an output information formation unit that acquires one or more pieces of advice information associated with a type identifier identifying the type determined by the second judgment unit, and forms output information that contains the type identifier and the one or more pieces of advice information, wherein the information output unit outputs the output information formed by the output information formation unit.

With such a configuration, it is possible to present an advance corresponding to the user's blood sugar constitution type.

A blood sugar constitution determination device according to an eighth aspect of the present invention is the blood sugar constitution determination device according to the first aspect of the invention, wherein the one or more pieces of question information in the storage unit are questions acquired by a simple questionnaire acquisition device, and the simple questionnaire acquisition device includes: an estimated type acquisition unit that acquires an estimated type identifier, using answers to M questions out of answers that are answers to N questions and that correspond to any actual type identifier of two or more actual type identifiers, where M is less than N; a question determination unit that, when an accuracy regarding a degree of matching between the estimated type identifier acquired by the estimated type acquisition unit and the actual type identifier satisfies a predetermined accuracy condition, determines M questions corresponding to the estimated type identifier; and a simple questionnaire output unit that outputs a simple questionnaire that is information regarding a question group that is a group of the M questions determined by the question determination unit.

With such a configuration, it is possible to very easily know the user's blood sugar constitution type.

A blood sugar constitution determination device according to a ninth aspect of the present invention is the blood sugar constitution determination device according to the second or third aspect of the invention, wherein the one or more first questions in the first question storage unit are questions acquired by a simple questionnaire acquisition device, and the simple questionnaire acquisition device includes: an estimated type acquisition unit that acquires estimated risk presence/absence information, using answers to M questions out of answers that are answers to N questions and that correspond to actual risk presence/absence information specifying presence or absence of the risk, where M is less than N; a question determination unit that, when an accuracy regarding a degree of matching between the estimated risk presence/absence information acquired by the estimated type acquisition unit and the actual risk presence/absence information satisfies a predetermined accuracy condition, determines M questions corresponding to the estimated risk presence/absence information; and a simple questionnaire output unit that outputs a simple questionnaire that is information regarding a question group that is a group of the M questions determined by the question determination unit.

With such a configuration, it is possible to very easily know the user's blood sugar constitution type.

A blood sugar constitution determination device according to a tenth aspect of the present invention is the blood sugar constitution determination device according to the second or third aspect of the invention, wherein one or more kinds of questions of the one or more second questions in the second question storage unit are questions acquired by a simple questionnaire acquisition device, and the simple questionnaire acquisition device includes: an estimated type acquisition unit that acquires an estimated type identifier, using answers to M questions out of answers that are answers to N questions and that correspond to any actual type identifier of two or more actual type identifiers, where M is less than N; a question determination unit that, when an accuracy regarding a degree of matching between the estimated type identifier acquired by the estimated type acquisition unit and the actual type identifier satisfies a predetermined accuracy condition, determines M questions corresponding to the estimated type identifier; and a simple questionnaire output unit that outputs a simple questionnaire that is information regarding a question group that is a group of the M questions determined by the question determination unit.

With such a configuration, it is possible to very easily know the user's blood sugar constitution type.

A blood sugar constitution determination device according to an eleventh aspect of the present invention is the blood sugar constitution determination device according to any one of the first to tenth aspects of the invention, wherein the blood sugar constitution type is a combination of a type of insulin sensitivity and a type of insulin secretion.

With such a configuration, it is possible to easily know of which of the four blood sugar constitution types the user is.

### Advantageous Effects of Invention

With the blood sugar constitution determination device according to the present invention, it is possible to easily know the user's blood sugar constitution type.

### Brief Description of Drawings

FIG. 1 is a conceptual diagram for a blood sugar constitution determination system A according to a first embodiment.
FIG. 2 is a block diagram for the blood sugar constitution determination system A according to the same.
FIG. 3 is a block diagram for a blood sugar constitution determination device 2 according to the same.
FIG. 4 is a flowchart illustrating a first operation example of a learning device 1 according to the same.
FIG. 5 is a flowchart illustrating a second operation example of the learning device 1 according to the same.
FIG. 6 is a flowchart illustrating a first operation example of the blood sugar constitution determination device 2 according to the same.
FIG. 7 is a flowchart illustrating a second operation example of the blood sugar constitution determination device 2 according to the same.
FIG. 8 is a flowchart illustrating an example of operation of a user terminal 3 according to the same.
FIG. 9 is a diagram showing a user information management table according to the same.
FIG. 10 is a diagram showing a first questionnaire according to the same.
FIG. 11 is a diagram showing a second questionnaire according to the same.
FIG. 12 is a diagram showing an example of output according to the same.
FIG. 13 is a block diagram for a simple questionnaire acquisition device B according to a second embodiment.
FIG. 14 is a flowchart illustrating an example of operation of the simple questionnaire acquisition device B according to the same.
FIG. 15 is a flowchart illustrating a first example of question determination processing according to the same.
FIG. 16 is a flowchart illustrating a first example of contribution acquisition processing according to the same.
FIG. 17 is a flowchart illustrating a second example of contribution acquisition processing according to the same.
FIG. 18 is a flowchart illustrating a second example of question determination processing according to the same.
FIG. 19 is a diagram showing a questionnaire according to the same.
FIG. 20 is a diagram showing answers according to the same.
FIG. 21 is an overview of a computer system according to the above-described embodiments.
FIG. 22 is a block diagram for the computer system according to the same.

### Description of Embodiments

Hereinafter, embodiments of a blood sugar constitution determination device and so on will be described with reference to the drawings. Note that components denoted by the same reference numerals in the embodiments perform similar operations, and therefore repetitive descriptions may be omitted.

### First Embodiment

In the present embodiment, a blood sugar constitution determination device that acquires and outputs a blood sugar constitution type, using answers to one or more questions. Note that the blood sugar constitution type is a type of health condition related to blood sugar. The blood sugar constitution type is, for example, one of four types, which are combinations of types of insulin sensitivity (either no resistance or a tendency to resistance) and types of insulin secretion (either normal or poor). The blood sugar constitution type is, for example, a type in which the blood sugar level rises easily or not, or a type in which the blood sugar level falls easily or not. The number, variations, etc. of blood sugar constitution types are not limited. It is preferable that the questionnaire includes questions regarding matters that are not considered to directly affect blood sugar levels. It is preferable that the questionnaire includes many questions that are easy to answer. Questions regarding lifestyle include, for example, questions regarding eating habits, questions regarding water or alcohol drinking habits, questions about exercise habits or lifestyle intensity, questions about sleep or sleepiness, questions about hygiene, and other general questions about lifestyle habits. General questions include, for example, questions about the frequency of brushing teeth, questions about the degree of use of stairs.

The present embodiment describes a blood sugar constitution determination device that detects the presence or absence of a risk related to blood sugar, using a first answer to each of one or more first questions included in a questionnaire including questions regarding lifestyle habits, and thereafter accepts a second answer to additional one or more second questions for a user having a risk, and acquires and outputs a blood sugar constitution type. That is to say, the present embodiment describes a blood sugar constitution determination apparatus that prompts users to answer questions in two stages, thereby enabling users who have no risk related to blood sugar to easily know the status of blood sugar.

Also, the present embodiment describes a blood sugar constitution determination device that acquires a blood sugar constitution type, using one or more second answers and one or more first answers.

Also, the present embodiment describes a blood sugar constitution determination device that acquires a blood sugar constitution type by performing machine learning prediction processing using a learning model for the acquisition of a blood sugar constitution type.

Also, the present embodiment describes a blood sugar constitution determination device that acquires and outputs advice information, using a blood sugar constitution type.

Also, the present embodiment describes a blood sugar constitution determination device that acquires and outputs advice information, using one or more answers in addition to a blood sugar constitution type.

Aso, the present embodiment describes a learning device capable of building a learning model that is used to predict a blood sugar constitution type.

Also, the present embodiment describes a blood sugar constitution determination system including a blood sugar constitution determination device that receives answers from a user terminal and transmits a blood sugar constitution type or the like to the user terminal. Note that the blood sugar constitution determination may be a standalone device.

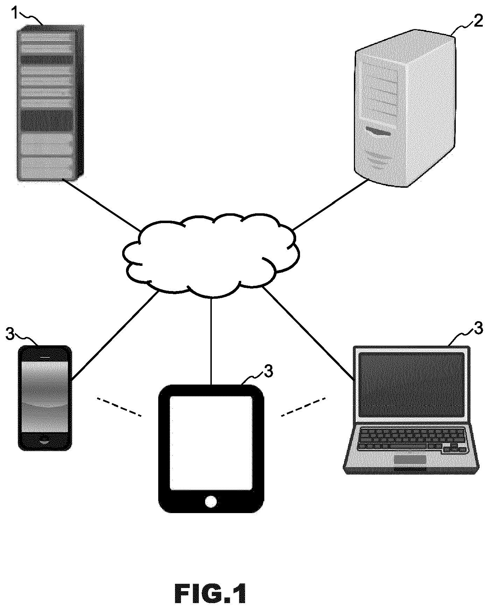

FIG. 1 is a conceptual diagram for a blood sugar constitution determination system A according to the present embodiment. The blood sugar constitution determination system A includes a learning device 1, a blood sugar constitution determination device 2, and one or more user terminals 3.

The learning device 1 is a device that acquires source information, which will be described later, using a type identifier identifying a blood sugar constitution type and answer information corresponding to two or more questions included in a questionnaire. The learning device 1 is, for example, a so-called personal computer, a tablet terminal, a smart phone, a server, or the like, and there is no limitation on the type thereof. The server is, for example, a cloud server or an ASP server, and there is no limitation on the type thereof. The learning device 1 may be a standalone device or a server that receives an instruction from the user terminals 3 to operate.

The blood sugar constitution determination device 2 is, for example, a server. The server is, for example, a cloud server or an ASP server, and there is no limitation on the type thereof. However, the blood sugar constitution determination device 2 may be a standalone device such as a so-called personal computer, a tablet terminal, a smart phone, or the like.

Each user terminal 3 is a terminal used by a user to acquire a blood sugar constitution type. Each user terminal 3 is a so-called personal computer, a tablet terminal, a smart phone, or the like, and there is no limitation on the type thereof.

The learning device 1, the blood sugar constitution determination device 2, and the user terminals 3 may be able to communicate with each other via the Internet, a LAN, or the like.

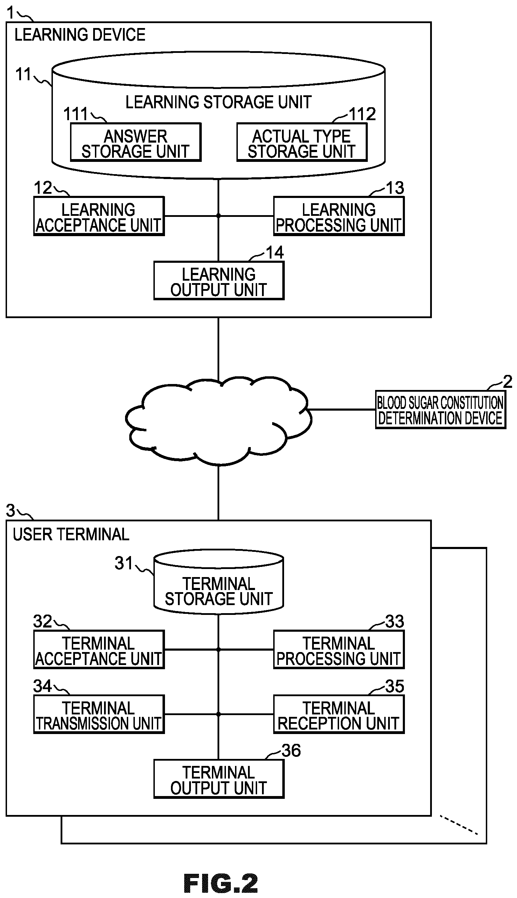

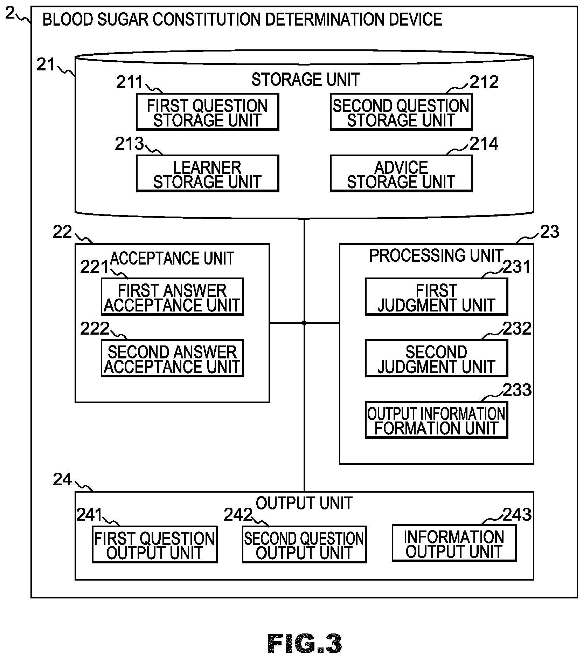

FIG. 2 is a block diagram for the blood sugar constitution determination system A according to the present embodiment. FIG. 3 is a block diagram for the blood sugar constitution determination device 2.

The learning device 1 includes a learning storage unit 11, a learning acceptance unit 12, a learning processing unit 13, and a learning output unit 14. The learning storage unit 11 includes an answer storage unit 111 and an actual type storage unit 112.

The blood sugar constitution determination device 2 includes a storage unit 21, an acceptance unit 22, a processing unit 23, and an output unit 24. The storage unit 21 includes a first question storage unit 211, a second question storage unit 212, a source information storage unit 213, and an advice storage unit 214. The acceptance unit 22 includes a first answer acceptance unit 221 and a second answer acceptance unit 222. The processing unit 23 includes a first judgment unit 231, a second judgment unit 232, and an output information formation unit 233. The output unit 24 includes a first question output unit 241, a second question output unit 242, and an information output unit 243.

Each user terminal 3 includes a terminal storage unit 31, a terminal acceptance unit 32, a terminal processing unit 33, a terminal transmission unit 34, a terminal reception unit 35, and a terminal output unit 36.

The learning storage unit 11 included in the learning device 1 stores various kinds of information. Examples of the various kinds of information include answer information and a type. The type may also be referred to as a type identifier. The type is, for example, "1", "2", "3", or "4". The type "1" is, for example, information indicating "normal" regarding insulin secretion and "no resistance" regarding insulin sensitivity. The type "2" is, for example, information indicating "normal" regarding insulin secretion and "tendency to resistance" regarding insulin sensitivity. The type "3" is, for example, information indicating "poor" regarding insulin secretion and "no resistance" regarding insulin sensitivity. The type "4" is, for example, information indicating "poor" regarding insulin secretion and "tendency to resistance" regarding insulin sensitivity. Note that the type "1" is a normal type. The types "2", "3", and "4" are abnormal types.

The answer storage unit 111 stores two or more answers. The answers may also be referred to as answer information. The answers may be information specifying the user's answers to questions. The answers are answers to questions. The answers are associated with actual types. The actual types are actual user types. The actual types may also be referred to as actual type identifiers. The answers are associated with actual risk presence/absence information. Actual risk presence/absence information is information indicating whether or not the user has a risk related to blood sugar.

The answer storage unit 111 stores, for example, answers associated with identifiers. The answers are the user's answers to the questions contained in the questionnaire. Note that users are respondents. The answers are typically associated with the question identifiers of the questions contained in the questionnaire. The question identifiers are information identifying the questions, such as a question numbers and question IDs. The identifiers are user identifiers, day identifiers, or user identifiers and day identifiers. User identifiers are information that identifies users, and are for example, IDs, e-mail addresses, telephone numbers, or the IDs of terminals used by the users (for example, IP addresses, MAC addresses, terminal identifiers, or the like).

Answers may be associated with user attribute values. User attribute values are attribute values of each user, such as the age, the height, the weight, the BMI, and the sex.

It is preferable that answers are information indicating answers to one of M questions (M is a natural number no less than 1) selected from N questions (N is a natural number no less than 2), provided by a simple questionnaire acquisition device B, which will be described later. Note that "N > M" is satisfied.

Note that the answer storage unit 111 may store two or more first answers and two or more second answers. The first answers are answers to the first questions that are used to judge the presence or absence of a risk related to blood sugar. The first answers are associated with risk presence/absence information specifying the presence or absence of a risk related to blood sugar. The second answers are answers to the second questions that are used to judge the blood sugar constitution type. The second answers are associated with the actual type identifiers in the case where there is a risk. The first answers and the second answers are both associated with identifiers, for example.

The actual type storage unit 112 stores two or more actual types. The actual types may also be referred to as actual type identifiers. The actual types are information identifying the actual blood sugar constitution types of the users who answered. Each actual type is typically associated with one or more answers. The actual types are typically associated with a group of answers to the questions contained in the questionnaire. The actual types are associated with identifiers, for example.

There is no limitation on the method for acquiring the actual types. The actual types are, for example, information acquired from a sugar tolerance test. The actual type identifiers are, for example, blood sugar constitution types determined in the following manner: after a user ingests 75 g of sugar, a blood sample is taken (for example, every 30 minutes) from 0 minutes to 120 minutes, the blood is tested, and the blood sugar level is measured to acquire changes in the user's blood sugar level over time, and a doctor, a system, or the like determines a blood sugar constitution type with reference to the changes over time. That is to say, the actual types may be information input manually.

The actual type storage unit 112 stores two or more pieces of actual risk presence/absence information. Actual risk presence/absence information is typically associated with one or more answers. Actual risk presence/absence information is typically associated with a group of answers to the questions contained in the questionnaire. Actual risk presence/absence information is, for example, associated with identifiers. Actual risk presence/absence information may be associated with actual types. Actual risk presence/absence information is information specifying the presence or absence of the risk related to the actual blood sugar of the users. Actual risk presence/absence information is typically associated with one or more first answers. Each actual type is typically associated with one or more second answers. Actual risk presence/absence information and the actual types are, for example, associated with identifiers.

The learning acceptance unit 12 accepts various kinds of instructions, information, and so on. Examples of the various kinds of instructions, information, and so on include a learning instruction. The learning instruction is an instruction to acquire source information. There is no limitation on the input means for inputting various kinds of instructions, information, and so on. For example, a touch panel, a keyboard, a mouse, a means using a menu screen, or the like may be employed. Note that the learning acceptance unit 12 may receive various kinds of instructions, information, and so on from the user terminals 3.

Source information is information that is used as the source for estimating the type based on the pieces of answer information corresponding to two or more questions. Source information is, for example, a learner, which will be described later. Source information is, for example, a correspondence table, which will be described later. Source information is typically acquired by the learning processing unit 13, which will be described later.

The learning processing unit 13 acquires source information. The processing by the learning processing unit 13 is, for example, (1) or (2) shown below. It is preferable that the learning processing unit 13 performs the processing (1) shown in below to acquire a learner.

### (1) In the case where source information is a learner

### (1-1) In the case where source information is a learner that determines a blood sugar constitution type by performing prediction processing once

The learning processing unit 13 acquires two or more sets from the learning storage unit 11. Each set is constituted by an actual type and a group of two or more answers. The actual type in the set and the two or more answers in the set are associated with each other. The actual type and the group of answers in the set are, for example, associated with the same identifier. Such set may also be referred to as training data.

The learning processing unit 13 performs learning processing according to a machine learning algorithm, using the acquired two or more sets, to acquire a learner. More specifically, for example, the learning processing unit 13 provides the acquired two or more sets to a module that performs machine learning processing, and executes the module to acquire a learner. Note that the learner may also be referred to as a classifier, a learning model, a model, or the like. Note that this learner is a learner that is to be provided to a module that performs machine learning prediction processing together with a group of two or more answers, to acquire a type.

Note that the module for machine learning processing may be a random forest, a decision tree, deep learning, an SVR, or the like, and there is no limitation on the algorithm thereof. In addition, for machine learning, it is possible to use various machine learning functions in the TensorFlow library, fastText, tinySVM, an R language random forest module, or the like, or various existing libraries, for example.

### (1-2) In the case where source information is a first learner that is used to judge the presence or absence of a risk related to blood sugar and a second learner for determining the blood sugar constitution type when there is a risk.

The learning processing unit 13 acquires two or more first sets from the learning storage unit 11. The first set here is constituted by actual risk presence/absence information and a group of one or more first answers. Actual risk presence/absence information indicates that there is a risk (for example, "1") or that there is no risk (for example, "0"). It is preferable that the first answers here are information indicating answers to one of M₁ questions (M₁ is a natural number no less than 1) selected from N₁ questions (N₁ is a natural number no less than 2), provided by the simple questionnaire acquisition device B, which will be described later. Note that N₁ > M₁ is satisfied.

Next, the learning processing unit 13 performs learning processing according to a machine learning algorithm, using the acquired two or more first sets, to acquire a first learner. Note that the learning processing may be the same processing as the above-described learning processing. The first learner is data used to perform prediction processing to acquire estimated risk presence/absence information upon accepting one or more first answers. Estimated risk presence/absence information is risk presence/absence information that has been estimated.

Also, the learning processing unit 13 acquires two or more second sets from the learning storage unit 11. Each second set here is constituted by an actual type and a group of one or more second answers. It is preferable that the first answers here are information indicating answers to one of M₂ questions (M₂ is a natural number no less than 1) selected from N₂ questions (N₂ is a natural number no less than 2), provided by the simple questionnaire acquisition device B, which will be described later. Note that N₂ > M₂ is satisfied.

Here, the learning processing unit 13 may acquire a second set and one or more first answers paired with the second set. It is preferable that the one or more first answers acquired here are some first answers of the first answers used to acquire the first learner.

Next, the learning processing unit 13 performs learning processing according to a machine learning algorithm, using the acquired two or more second sets, to acquire a second learner. It is preferable that the learning processing unit 13 performs learning processing according to a machine learning algorithm, using two or more sets each consisting of a second set and one or more first answers paired with the second set, to acquire the second learner.

Note that the learning processing may be the same processing as the above-described learning processing. The second learner is data used to perform prediction processing to acquire estimated type upon accepting one or more second answers. Note that the estimated type is a type that has been estimated.

The first algorithm of machine learning for acquiring the first learner and the second algorithm of machine learning for acquiring the second learner may be the same or different. For example, the first algorithm is the SVM, and the second algorithm is the random forest or deep learning.

### (2) In the case where source information is a correspondence table

### (2-1) In the case where source information is one correspondence table that determines the blood sugar constitution type.

The learning processing unit 13 acquires two or more sets from the learning storage unit 11. Each set is constituted by an actual type and a group of two or more answers.

Thereafter, for each set, the learning processing unit 13 acquires a vector whose elements are two or more answers. Next, for each set, the learning processing unit 13 acquires correspondence information indicating the correspondence between the vector and the actual type. Next, the learning processing unit 13 builds a correspondence table containing, as records, correspondence information for each of the two or more sets. Note that correspondence information contains, for example, a vector and an actual type. Correspondence information contains, for example, a link to a vector and a link to an actual type.

### (2-2) In the case where source information is a first correspondence table that is used to judge the presence or absence of a risk related to blood sugar and a second correspondence table for determining the blood sugar constitution type when there is a risk.

The learning processing unit 13 acquires two or more first sets from the learning storage unit 11. The first set here is constituted by risk presence/absence information specifying the presence or absence of a risk, and a group of two or more first answers.

Thereafter, for each set, the learning processing unit 13 acquires a vector whose elements are two or more first answers. Next, for each set, the learning processing unit 13 acquires first correspondence information indicating the correspondence between the vector and risk presence/absence information. Next, the learning processing unit 13 builds a first correspondence table containing, as records, first correspondence information for each of the two or more first sets. Note that the first correspondence information has, for example, the same data structure as the above-described correspondence information.

Also, the learning processing unit 13 acquires two or more second sets from the learning storage unit 11. Each second set here is constituted by an actual type and a group of one or more second answers. It is preferable that the learning processing unit 13 acquires two or more sets each consisting of a second set and one or more first answers paired with the second set.

Thereafter, for each second set, the learning processing unit 13 acquires a vector whose elements are two or more second answers. Here, it is preferable that the learning processing unit 13 acquires a vector whose elements are the two or more second answers contained in the two or more sets and the one or more first answers.

Next, for each second set, the learning processing unit 13 acquires second correspondence information indicating the correspondence between the vector and the actual type. Next, the learning processing unit 13 builds a second correspondence table containing, as records, second correspondence information for each of the two or more second sets. Note that second correspondence information contains, for example, a vector and an actual type. Second correspondence information contains, for example, a link to a vector and a link to an actual type.

The learning output unit 14 outputs information. The learning output unit 14 outputs, for example, the source information acquired by the learning processing unit 13. Here, "output" typically means accumulation on a recording medium, but may be a concept that encompasses displaying on a display screen, projection using a projector, printing by a printer, the output of a sound, transmission to an external device, delivery of a processing result to another processing device or another program, and the like.

It is preferable that the learning output unit 14 accumulates source information in the source information storage unit 213 of the blood sugar constitution determination device 2.

The storage unit 21 included in the blood sugar constitution determination device 2 stores various kinds of information. Examples of the various kinds of information include two or more questions, one or more first questions, which will be described later, one or more second questions, which will be described later, one or more pieces of source information, and one or more pieces of user information. Note that the two or more questions may be referred to as a questionnaire. The one or more first questions may also be referred to as a first questionnaire. The one or more second questions may also be referred to as a second questionnaire.

Questions are question information for estimating the type. It is preferable that the two or more questions include a question regarding lifestyle. The questions may be the first questions described later or the second questions described later.

The first question storage unit 211 stores one or more first questions. The first questions are questions that are used to judge the presence or absence of a risk related to blood sugar. It is preferable that the one or more first questions include one or more questions regarding lifestyle.

The second question storage unit 212 stores one or more second questions. The second questions are questions for judging the blood sugar constitution type. It is preferable that the one or more second questions include one or more questions regarding lifestyle.

It is preferable that the blood sugar constitution type is a combination of the type of insulin sensitivity and the type of insulin secretion. The type of insulin sensitivity is, for example, either "no resistance" or "tendency to resistance". The type of insulin sensitivity is, for example, either "normal" or "poor".

The source information storage unit 213 stores one or more pieces of source information. Source information is typically information acquired by the learning device 1. Source information is, for example, the above-described learner, the above-described first learner, the above-described second learner, the above-described correspondence table, the above-described first correspondence table, or the above-described second correspondence table.

The advice storage unit 214 stores one or more pieces of advice information in association with one or more type identifiers identifying the blood sugar constitution type. There is no limitation on the content of advice information. The advice storage unit 214 may store one or more pieces of advice information in association with risk presence/absence information.

The acceptance unit 22 accepts various kinds of instructions, information, and so on. Examples of the various kinds of instructions, information, and so on include answers, the first answers, and the second answers. For example, the acceptance unit 22 accepts answers to two or more questions.

The "acceptance" here typically means reception from the user terminals 3, but may be a concept that includes acceptance of information input from an input device such as a keyboard, a mouse, or a touch panel, or acceptance of information read from a recording medium such as an optical disk, a magnetic disk, or a semiconductor memory.

The first answer acceptance unit 221 accepts first answers to one or more first questions from users. For example, the first answer acceptance unit 221 receives one or more first answers from a user terminal 3.

The second answer acceptance unit 222 accepts second answers to one or more second questions from users.

The processing unit 23 performs various kinds of processing. The processing unit 23 performs the processing by the first judgment unit 231, the second judgment unit 232, and the output information formation unit 233.

For example, the processing unit 23 or a judgment unit (not shown) acquires a type, using the one or more answers accepted by the acceptance unit 22.

For example, the processing unit 23 or a judgment unit (not shown) acquires a type by performing machine learning prediction processing, using the one or more answers accepted by the acceptance unit 22, and the learner. More specifically, for example, the processing unit 23 or a judgment unit (not shown) provides the one or more answers accepted by the acceptance unit 22 and the learner to a module that performs machine learning prediction processing, and executes the module to acquire a type. Note that the module for machine learning prediction processing may be a random forest, a decision tree, deep learning, an SVM, or the like, and there is no limitation on the algorithm thereof. In addition, for machine learning, it is possible to use various machine learning functions in the TensorFlow library, fastText, tinySVM, an R language random forest module, or the like, or various existing libraries, for example. Note that the learner is the learner acquired by the learning device 1.

For example, the processing unit 23 or a judgment unit (not shown) acquires a type, using the one or more answers accepted by the acceptance unit 22, and a correspondence table. More specifically, for example, the processing unit 23 or a judgment unit (not shown) forms a vector whose elements are answers to the one or more answers accepted by the acceptance unit 22. Next, the processing unit 23 or a judgment unit (not shown) searches the correspondence table for a vector that is closest to the vector, and acquires the type paired with the vector that is closest to the vector from the correspondence table.

The first judgment unit 231 judges whether or not the user has a risk related to blood sugar, using the one or more first answers accepted by the first answer acceptance unit 221.

For example, the first judgment unit 231 performs machine learning prediction processing, using the one or more first answers accepted by the first answer acceptance unit 221, and the first learner, to acquire estimated risk presence/absence information. More specifically, for example, the first judgment unit 231 provides the one or more first answers accepted by the first answer acceptance unit 221 and the first learner to the module that performs machine learning prediction processing, and executes the module to acquire estimated risk presence/absence information.

For example, the first judgment unit 231 forms a vector whose elements are the one or more first answers accepted by the first answer acceptance unit 221. Next, for example, the first judgment unit 231 searches the first correspondence table for a vector that is closest to the vector, and acquires the risk presence/absence information paired with the vector that is closest to the vector from the first correspondence table. Note that such risk presence/absence information is estimated risk presence/absence information.

The second judgment unit 232 determines the user's blood sugar constitution type, using the one or more second answers accepted by the second answer acceptance unit 222.

For example, the second judgment unit 232 performs machine learning prediction processing, using the one or more second answers accepted by the second answer acceptance unit 222 and the second learner to acquire an estimated type. More specifically, for example, the second judgment unit 232 provides the one or more second answers accepted by the second answer acceptance unit 222 and the second learner to a module that performs machine learning prediction processing, and executes the module to acquire an estimated type.

For example, the second judgment unit 232 forms a vector whose elements are the one or more second answers accepted by the second answer acceptance unit 222. Next, for example, the second judgment unit 232 searches the second correspondence table for the vector closest to the vector, and acquires the type paired with the vector that is closest to the vector from the second correspondence table. Note that such a type is an estimated type.

It is preferable that the second judgment unit 232 additionally uses the one or more first answers accepted by the first answer acceptance unit 221 to determine the user's blood sugar constitution type. It is preferable that the one or more first answers in such a case are some first answers of the first answers used to acquire risk presence/absence information.

For example, the second judgment unit 232 performs machine learning prediction processing, using the one or more second answers accepted by the second answer acceptance unit 222, the one or more first answers accepted by the first answer acceptance unit 221, and the second learner, to acquire an estimated type. More specifically, for example, the second judgment unit 232 provides the one or more second answers accepted by the second answer acceptance unit 222, the one or more first answers accepted by the first answer acceptance unit 221, and the second learner, to a module that performs machine learning prediction processing, and executes the module to acquire an estimated type.

For example, the second judgment unit 232 forms a vector whose elements are the one or more second answers accepted by the second answer acceptance unit 222 and the one or more first answers accepted by the first answer acceptance unit 221. Next, for example, the second judgment unit 232 searches the second correspondence table for the vector closest to the vector, and acquires the type paired with the vector that is closest to the vector from the second correspondence table. Note that such a type is an estimated type.

The output information formation unit 233 acquires one or more pieces of advice information associated with the type identifier identifying the type determined by the second judgment unit 232 from the advice storage unit 214, and forms output information having the type identifier and the one or more pieces of advice information.

The output unit 24 outputs various kinds of information. Examples of the various kinds of information include questions, the first questions, the second questions, and output information.

Here, "output" typically means transmission to the user terminals 3, but may be a concept that encompasses displaying on a display screen, projection using a projector, printing by a printer, the output of a sound, accumulation on a recording medium, delivery of a processing result to another processing device or another program, and the like.

The first question output unit 241 outputs one or more first questions from the first question storage unit 211.

The second question output unit 242 outputs one or more second questions from the second question storage unit 212 if the first judgment unit 231 judges that there is a risk.

The information output unit 243 outputs output information regarding the blood sugar constitution type. For example, the information output unit 243 outputs output information containing the one or more pieces of advice information associated with the estimated type determined by the second judgment unit 232. For example, the information output unit 243 outputs output information formed by the output information formation unit 233.

The terminal storage unit 31 included in each user terminal 3 stores various kinds of information. Examples of the various kinds of information include a user identifier and one or more user attribute values.

The terminal acceptance unit 32 accepts various kinds of instructions, information, and so on. Examples of the various kinds of instructions and information include one or more answers and a questionnaire output instruction. The answers may be the first answers or the second answers. The questionnaire output instruction is an instruction to output a questionnaire. It is preferable that the questionnaire is a simple questionnaire created by simple questionnaire acquisition device B, which will be described later.

There is no limitation on the input means for inputting various kinds of instructions and information. For example, a touch panel, a keyboard, a mouse, a means using a menu screen, or the like may be employed.

The terminal processing unit 33 performs various kinds of processing. Examples of the various kinds of processing include forming the instructions, information, and so on accepted by the terminal acceptance unit 32 into data structures of instructions, information, and so on to be transmitted. Examples of the various kinds of processing include forming the information received by the terminal reception unit 35 into a data structure to be output.

The terminal transmission unit 34 transmits various kinds of instructions, information, and so on to the blood sugar constitution determination device 2. The terminal transmission unit 34 transmits the instructions, information, and so on formed by the terminal processing unit 33, to the blood sugar constitution determination device 2.

The terminal reception unit 35 receives various kinds of information and so on. For example, the terminal reception unit 35 receives a questionnaire, estimated risk presence/absence information, an estimated type, advice information, or output information from the blood sugar constitution determination device 2.

The terminal output unit 36 outputs various kinds of information and so on. For example, the terminal output unit 36 outputs a questionnaire, estimated risk presence/absence information, an estimated type, advice information, or output information.

It is preferable that the learning storage unit 11, the answer storage unit 111, the actual type storage unit 112, the storage unit 21, the first question storage unit 211, the second question storage unit 212, the source information storage unit 213, the advice storage unit 214, and the terminal storage unit 31 are realized using a non-volatile recording medium, but they may be realized using a volatile recording medium.

There is no limitation on the process in which information is stored in the learning storage unit 11 and so on. For example, information may be stored in the learning storage unit 11 or the like via a recording medium, or information transmitted via a communication line or the like may be stored in the learning storage unit 11 or the like, or information input via an input device may be stored in the learning storage unit 11 or the like.

The learning acceptance unit 12 and the terminal acceptance unit 32 can be realized using a device driver for the input means such as a touch panel or a keyboard, or control software or the like for controlling the menu screen. The learning acceptance unit 12 may be realized using a wireless or wired communication means.

The learning processing unit 13, the learning output unit 14, the processing unit 23, the first judgment unit 231, the second judgment unit 232, the output information formation unit 233, and the terminal processing unit 33 can typically be realized using a processor, a memory, and so on. The processing procedures performed by the learning processing unit 13 and so on are typically realized using software, and the software is recorded on a recording medium such as a ROM. However, such processing procedures may be realized using hardware (a dedicated circuit). The processor is, for example, a CPU, an MPU, a GPU, or the like, and there is no limitation on the type thereof.

Note that the learning output unit 14 may be realized using a wireless or wired communication means.

The acceptance unit 22, the first answer acceptance unit 221, the second answer acceptance unit 222, and the terminal reception unit 35 are typically realized using a wireless or wired communication means, but may be realized using a broadcast receiving means.

The output unit 24, the first question output unit 241, the second question output unit 242, the information output unit 243, and the terminal transmission unit 34 are typically realized using a wireless or wired communication means, but may be realized using a broadcast means.

The terminal acceptance unit 32 can be realized using a device driver for the input means such as a touch panel or a keyboard, or control software or the like for controlling the menu screen.

The terminal output unit 36 may be regarded as including or not including an output device such as a display or a speaker. The terminal output unit 36 can be realized using the driver software of the output device, the driver software of the output device and the output device, or the like.

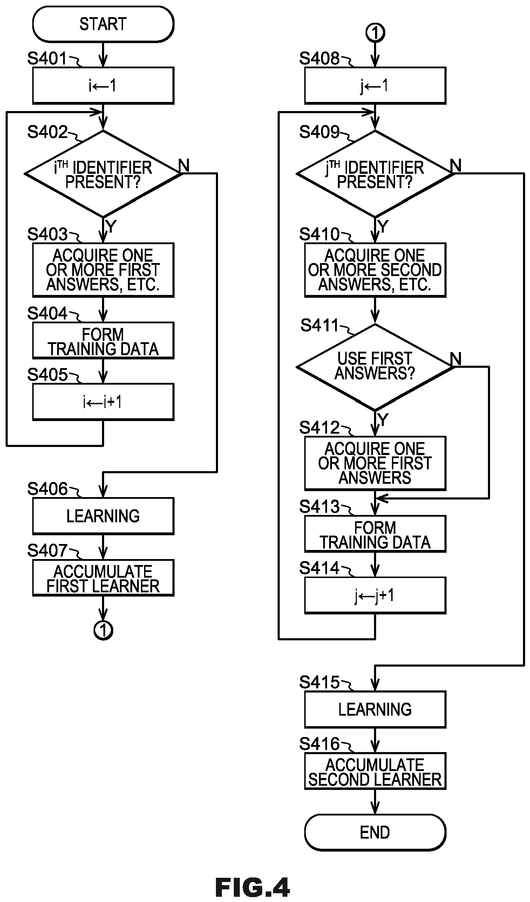

Next, examples of operation of the blood sugar constitution determination system A will be described. First, a first operation example of the learning device 1 will be described with reference to the flowchart shown in FIG. 4. The first operation example is an example of an operation performed to form two learners, namely the first learner and the second learner.

(Step S401) The learning processing unit 13 substitutes 1 for a counter i.

(Step S402) The learning processing unit 13 judges whether or not an i^{th} identifier is present. If the i^{th} identifier is present, processing proceeds to step S403, and if the i^{th} identifier is not present, processing proceeds to step S406. Note that the identifier is associated with one or more first answers and one or more second answers.

(Step S403) The learning processing unit 13 acquires a group of first answers paired with the i^{th} identifier and risk presence/absence information paired with the group of first answers, from the learning storage unit 11. Note that the learning processing unit 13 may determine and acquire risk presence/absence information based on the type. For example, the type identifier "1" indicates "risk presence/absence information "absence "0"", and the type identifiers "2", "3,", and "4" indicate risk presence/absence information "present "1"".

(Step S404) The learning processing unit 13 forms an i^{th} piece of training data, using the i^{th} group of first answers and the risk presence/absence information acquired in step S403, and accumulates it in a buffer (not shown). For example, the learning processing unit 13 forms an i^{th} piece of training data that is a vector whose elements are the first answers in the i^{th} group and the risk presence/absence information acquired in step S403.

(Step S405) The learning processing unit 13 increments the counter i by 1. The processing returns to step S402.

(Step S406) The learning processing unit 13 acquires the two or more pieces of training data accumulated in step S404 from the buffer (not shown), and acquires the first learner by performing machine learning prediction processing, using the two or more pieces of training data.

(Step S407) The learning processing unit 13 accumulates the first learner acquired in step S406 in the learning storage unit 11.

(Step S408) The learning processing unit 13 substitutes 1 for a counter j.

(Step S409) The learning processing unit 13 judges whether or not a j^{th} identifier is present. If the j^{th} identifier is present, processing proceeds to step S410, and if the j^{th} identifier is not present, processing proceeds to step S415. Note that the identifier is associated with one or more first answers and one or more second answers.

(Step S410) The learning processing unit 13 acquires a group of second answers paired with the j^{th} identifier, and an actual type paired with the group of second answers. Note that the group of second answers is present in the answer storage unit 111. The actual type is present in the actual type storage unit 112.

(Step S411) To form the second learner, the learning processing unit 13 judges whether or not to use one or more first answers. If the first answers are to be used, processing proceeds to step S412, and if the first answers are not to be used, processing proceeds to step S413. Note that whether or not to use the first answers is typically determined in advance.

(Step S412) The learning processing unit 13 acquires one or more first answers that are paired with the j^{th} identifier and that are to be used to form the second learner, from the answer storage unit 111. Note that the one or more first answers used to form the second learner are typically determined in advance.

(Step S413) The learning processing unit 13 forms the j^{th} piece of training data, using the j^{th} group of second answers and the actual type acquired in step S410, and accumulates it in the buffer (not shown), or forms the j^{th} piece of training data, using the j^{th} group of second answers acquired in step S410, the one or more pieces of first answers acquired in step S412, and the actual type, and accumulates it in the buffer (not shown). For example, the learning processing unit 13 forms the j^{th} piece of training data that is a vector whose elements are the answers and the actual type, and accumulates it in the buffer (not shown).

(Step S414) The learning processing unit 13 increments the counter j by 1. The processing returns to step S409.

(Step S415) The learning processing unit 13 acquires the two or more pieces of training data accumulated in step S413 from the buffer (not shown), and acquires the second learner by performing machine learning prediction processing, using the two or more pieces of training data.

(Step S416) The learning processing unit 13 accumulates the second learner acquired in step S415 in the learning storage unit 11. Processing is terminated.

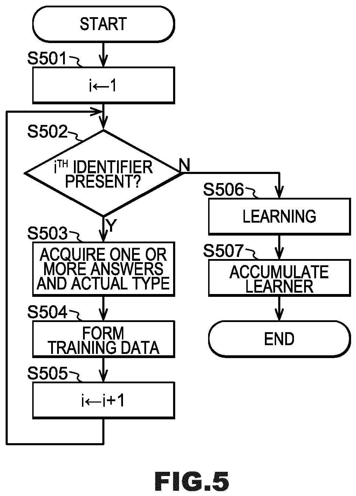

Next, a second operation example of the learning device 1 will be described with reference to the flowchart shown in FIG. 5. The second operation example is an example of an operation performed to form one learner. Such a learner is a learner for estimating a type.

(Step S501) The learning processing unit 13 substitutes 1 for the counter i.

(Step S502) The learning processing unit 13 judges whether or not the i^{th} identifier is present. If the i^{th} identifier is present, processing proceeds to step S403, and if the i^{th} identifier is not present, processing proceeds to step S406. Note that the identifier is associated with a group of one or more pieces of answer information.

(Step S503) The learning processing unit 13 acquires a group of answers paired with the i^{th} identifier, and an actual type paired with the group of answers.

(Step S504) The learning processing unit 13 forms the i^{th} piece of training data, using the i^{th} group of answers and the actual type acquired in step S503, and accumulates it in the buffer (not shown).

(Step S505) The learning processing unit 13 increments the counter i by 1. The processing returns to step S402.

(Step S506) The learning processing unit 13 acquires the two or more pieces of training data accumulated in step S504 from the buffer (not shown), and acquires the learner by performing machine learning prediction processing, using the two or more pieces of training data.

(Step S507) The learning processing unit 13 accumulates the learner acquired in step S506 in the learning storage unit 11. Processing is terminated.

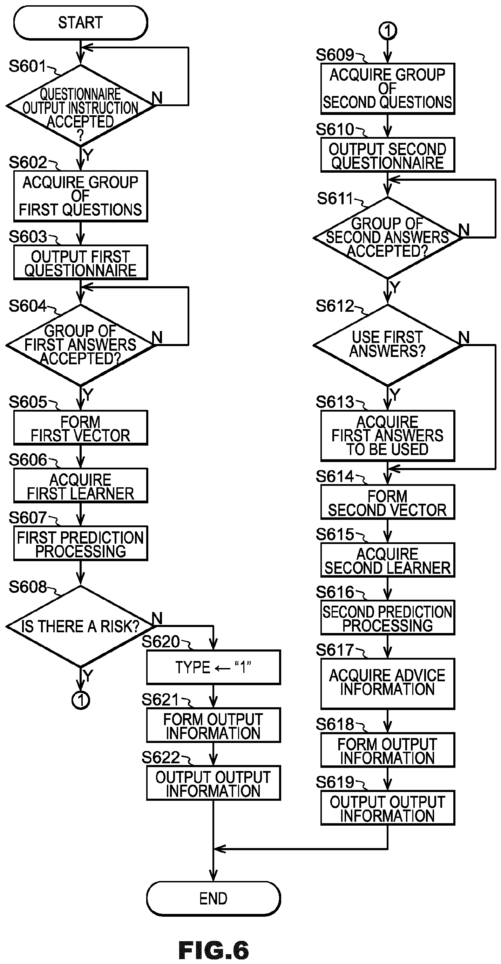

Next, a first operation example of the blood sugar constitution determination device 2 will be described with reference to the flowchart shown in FIG. 6.

(Step S601) The acceptance unit 22 judges whether or not a questionnaire output instruction has been accepted. If a questionnaire output instruction has been accepted, processing proceeds to step S602, and if a questionnaire output instruction has not been accepted, processing returns to step S601. Here, for example, the acceptance unit 22 judges whether or not a questionnaire output instruction has been received from a user terminal 3.

(Step S602) The processing unit 23 acquires a first questionnaire from the first question storage unit 211.

(Step S603) The first question output unit 241 outputs the first questionnaire acquired in step S602. Here, for example, the first question output unit 241 outputs the first questionnaire to the user terminal 3.

(Step S604) The first answer acceptance unit 221 judges whether or not a group of one or more first answers has been accepted. If a group of one or more first answers has been accepted, processing proceeds to step S605, and if a group of one or more first answers has not been accepted, processing returns to step S604. Here, for example, the first answer acceptance unit 221 judges whether or not a group of one or more first answers has been received from a user terminal 3.

(Step S605) The first judgment unit 231 forms a first vector that is a vector whose elements are the first answers, using the group of one or more first answers accepted in step S604.

(Step S606) The first judgment unit 231 acquires the first learner from the source information storage unit 213.

(Step S607) The first judgment unit 231 acquires estimated risk presence/absence information by performing first prediction processing, which is machine learning prediction processing, using the first vector acquired in step S605 and the first learner acquired in step S606.

(Step S608) The first judgment unit 231 judges whether or not the estimated risk presence/absence information acquired in step S607 indicates that there is a risk or there is no risk. If there is a "risk", processing proceeds to step S609, and if there is "no risk", processing proceeds to step S620.

(Step S609) The processing unit 23 acquires a second questionnaire from the second question storage unit 212.

(Step S610) The second question output unit 242 outputs the second questionnaire acquired in step S609. Here, for example, the second question output unit 242 outputs the second questionnaire to the user terminal 3.

(Step S611) The second answer acceptance unit 222 judges whether or not a group of one or more second answers has been accepted. If a group of second answers has been accepted, processing proceeds to step S612, and if a group of second answers has not been accepted, processing returns to step S611. Here, for example, the second answer acceptance unit 222 judges whether or not a group of one or more second answers has been received from a user terminal 3.

(Step S612) The second judgment unit 232 judges whether or not to use the one or more first answers in the second prediction processing. If the first answers are to be used, processing proceeds to step S613, and if the first answers are not to be used, processing proceeds to step S614.

(Step S613) The second judgment unit 232 acquires one or more first answers to be used in the second prediction processing of the first answers accepted in step S604.

(Step S614) The second judgment unit 232 forms a second vector. Note that the second vector is a vector whose elements are the one or more second answers, or a vector whose elements are the one or more second answers and the one or more first answers acquired in step S613.

(Step S615) The second judgment unit 232 acquires the second learner from the source information storage unit 213.

(Step S616) The second judgment unit 232 acquires an estimated type identifier by performing second prediction processing, which is machine learning prediction processing, using the second vector acquired in step S614 and the second learner acquired in step S615.

(Step S617) The output information formation unit 233 acquires advice information paired with the estimated type acquired in step S616, from the advice storage unit 214.

(Step S618) The output information formation unit 233 forms output information, using the estimated type identifier acquired in step S616 and the advice information acquired in step S617.

(Step S619) The information output unit 243 outputs the output information formed in step S618. Processing is terminated. Here, for example, the information output unit 243 transmits the output information to the user terminal 3.

(Step S620) The first judgment unit 231 substitutes information ("1" here) indicating that there is "no risk", for the variable "type".

(Step S621) The output information formation unit 233 forms output information using the identifier "1" acquired in step S620.

(Step S622) The information output unit 243 outputs the output information formed in step S621. Processing is terminated. Here, for example, the information output unit 243 transmits the output information to the user terminal 3.

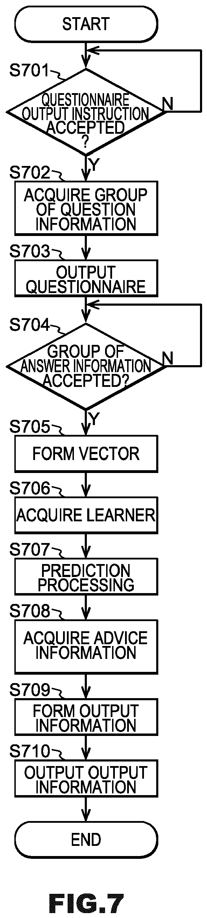

Next, a second operation example of the blood sugar constitution determination device 2 will be described with reference to the flowchart shown in FIG. 7.

(Step S701) The acceptance unit 22 judges whether or not a questionnaire output instruction has been accepted. If a questionnaire output instruction has been accepted, processing proceeds to step S702, and if a questionnaire output instruction has not been accepted, processing returns to step S701. Here, for example, the acceptance unit 22 judges whether or not a questionnaire output instruction has been received from a user terminal 3.

(Step S702) The processing unit 23 acquires a questionnaire from the storage unit 21. Note that the questionnaire is a group of answers.

(Step S703) The output unit 24 outputs the questionnaire acquired in step S702. Here, for example, the output unit 24 outputs the questionnaire to the user terminal 3.

(Step S704) The acceptance unit 22 judges whether or not a group of one or more pieces of answer information has been accepted. If a group of answers has been accepted, processing proceeds to step S705, and if a group of answers has not been accepted, processing returns to step S704. Here, for example, the acceptance unit 22 judges whether or not a group of one or more answers has been received from a user terminal 3.

(Step S705) The processing unit 23 (or a judgment unit (not shown)) forms a vector whose elements are answers, using the group of one or more answers accepted in step S704.

(Step S706) The processing unit 23 (or the judgment unit (not shown)) acquires the learner from the source information storage unit 213.

(Step S707) The processing unit 23 (or the judgment (not shown)) acquires an estimated type by performing machine learning prediction processing, using the vector acquired in step S705 and the learner acquired in step S706.

(Step S708) The output information formation unit 233 acquires advice information paired with the estimated type acquired in step S707, from the advice storage unit 214.

(Step S709) The output information formation unit 233 forms output information, using the estimated type acquired in step S707 and the advice information acquired in step S708.

(Step S710) The information output unit 243 outputs the output information formed in step S709. Processing is terminated. Here, for example, the information output unit 243 transmits the output information to the user terminal 3.

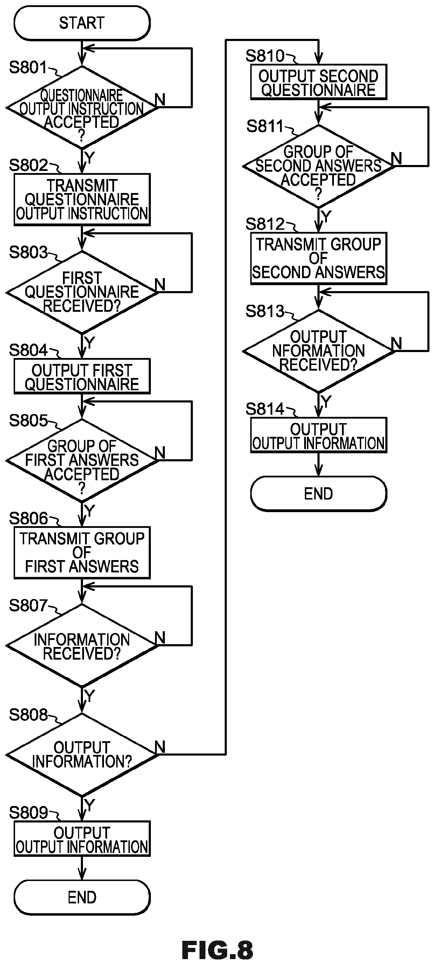

Next, an operation example of a user terminal 3 will be described with reference to the flowchart in FIG. 8.

(Step S801) The terminal acceptance unit 32 judges whether or not a questionnaire output instruction has been accepted. If a questionnaire output instruction has been accepted, processing proceeds to step S802, and if a questionnaire output instruction has not been accepted, processing returns to step S801.

(Step S802) The terminal processing unit 33 forms a questionnaire output instruction to be transmitted. The terminal transmission unit 34 transmits the formed questionnaire output instruction to the blood sugar constitution determination device 2.

(Step S803) The terminal reception unit 35 judges whether or not a first questionnaire has been received. If a first questionnaire has been received, processing proceeds to step S804, and if a first questionnaire has not been received, processing returns to step S803.

(Step S804) The terminal processing unit 33 forms a first questionnaire to be output, based on the first questionnaire received in step S803. The terminal output unit 36 outputs the first questionnaire.

(Step S805) The terminal acceptance unit 32 judges whether or not a group of first answers has been accepted from the user. If a group of first answers has been accepted, processing proceeds to step S806, and if a group of first answers has not been accepted, processing returns to step S805.

(Step S806) The terminal processing unit 33 forms a group of first answers to be transmitted. The terminal transmission unit 34 transmits the formed group of first answers to the blood sugar constitution determination device 2.

(Step S807) The terminal reception unit 35 judges whether or not information has been received from the blood sugar constitution determination device 2. If information has been received, processing proceeds to step S808, and if information has not been received, processing returns to step S807.

(Step S808) The terminal processing unit 33 judges whether or not the information received in step S807 is output information. If the information is output information, processing proceeds to step S809, and if the information is not output information, processing proceeds to step S810. Note that the information received in step S807 is output information or second questionnaire.

(Step S809) The terminal output unit 36 outputs the output information received in step S807. Processing is terminated.

(Step S810) The terminal output unit 36 outputs the second questionnaire received in step S807.

(Step S811) The terminal acceptance unit 32 judges whether or not a group of second answers has been accepted from the user. If a group of second answers has been accepted, processing proceeds to step S812, and if a group of second answers has not been accepted, processing returns to step S811.

(Step S812) The terminal processing unit 33 forms a group of second answers to be transmitted. The terminal transmission unit 34 transmits the formed group of second answers to the blood sugar constitution determination device 2.

(Step S813) The terminal reception unit 35 judges whether or not output information has been received from the blood sugar constitution determination device 2. If output information has been received, processing proceeds to step S814, and if output information has not been received, processing returns to step S813.

(Step S814) The terminal output unit 36 outputs the received output information. Processing is terminated.

Note that, in the flowchart in FIG. 8, the user terminal 3 may accept answers to the two or more questions included in the questionnaire, transmit the group of answers to the blood sugar constitution determination device 2, and receive output information including a type from the blood sugar constitution determination device 2 in response to the transmission.

Hereinafter, a specific operation example of the blood sugar constitution determination system A according to the present embodiment will be described.

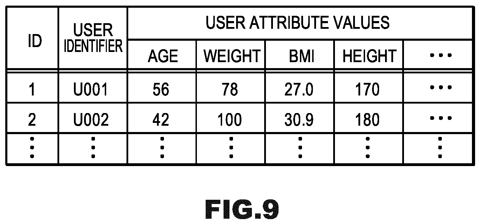

Now, it is assumed that the storage unit 21 of the blood sugar constitution determination device 2 stores the user information management table shown in FIG. 9. The user information management table is a table for managing one or more pieces of user information. The user information management table stores one or more records each containing "ID", "user identifier", and "user attribute values".

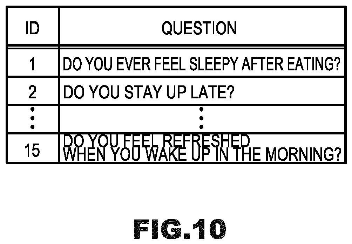

The first question storage unit 211 stores the first questionnaire shown in FIG. 10. The first questionnaire contains fifteen first questions. It is also assumed that the first questionnaire is a simple questionnaire acquired by a simple questionnaire acquisition device B, which will be described later. The fifteen first questions are questions corresponding to the one or more first answers used to acquire the first learner.

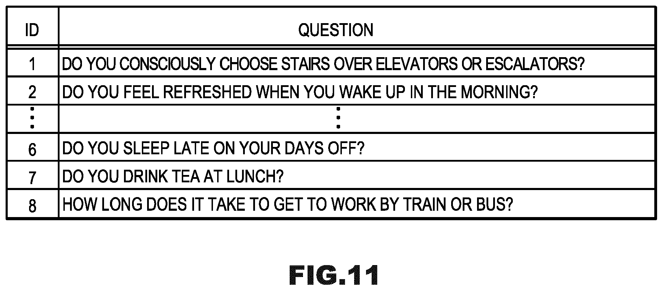

It is also assumed that the second question storage unit 212 stores the second questionnaire shown in FIG. 11. The second questionnaire contains eight second questions. It is also assumed that the second questionnaire is a simple questionnaire acquired by the simple questionnaire acquisition device B, which will be described later. The eight second questions are questions corresponding to the one or more second answers used to acquire the second learner.

It is also assumed that the source information storage unit 213 stores the first learner and the second learner. Here, the first learner is a model that is used to estimate risk presence/absence information, using one or more user attribute values and one or more first answers. Here, the second learner is a model that is used to estimate a type of a person with a risk, using one or more user attribute values, one or more first answers, and one or more second answers.

It is assumed that the learning processing unit 13 of the learning device 1 has performed machine learning processing to acquire the first learner, using two or more pieces of training data each containing one or more user attribute values and one or more first answers, and has accumulated the first learner in the source information storage unit 213 of the blood sugar constitution determination device 2.

It is also assumed that the learning processing unit 13 of the learning device 1 has performed machine learning processing to acquire the second learner, using two or more pieces of training data each containing one or more user attribute values, one or more first answers, and one or more second answers, and has accumulated the second learner in the source information storage unit 213 of the blood sugar constitution determination device 2.

Here, it is assumed that the one or more first answers used to acquire the second learner are some answers of the one or more first answers used to acquire the first learner.

Furthermore, it is assumed that the first questions corresponding to the one or more first answers and the second questions corresponding to the one or more second answers are both questions determined by the simple questionnaire acquisition device B described later, from the same large number of questions (for example, 350 questions).

It is also assumed that the advice storage unit 214 stores advice information in association with types.

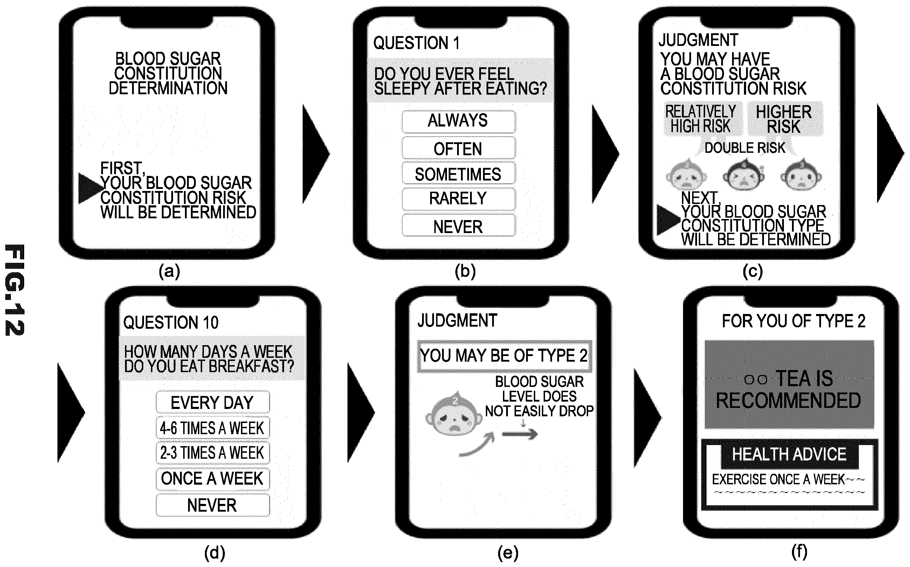

In such a situation, it is assumed that the user A with the user identifier "U001" inputs a questionnaire output instruction to their own user terminal 3 (for example, see FIG. 12(a)). Next, the terminal acceptance unit 32 accepts the questionnaire output instruction. Thereafter, the terminal processing unit 33 forms a questionnaire output instruction to be transmitted. Next, the terminal transmission unit 34 transmits the formed questionnaire output instruction to the blood sugar constitution determination device 2.

Next, the acceptance unit 22 of the blood sugar constitution determination device 2 receives the questionnaire output instruction. The processing unit 23 acquires a first questionnaire containing fifteen first questions (see FIG. 10) from the first question storage unit 211. Next, the first question output unit 241 outputs the first questionnaire to the user terminal 3.

Next, the terminal reception unit 35 of the user terminal 3 receives the first questionnaire. The terminal processing unit 33 forms a first questionnaire to be output, based on the received first questionnaire. The terminal output unit 36 outputs the first questionnaire. An example of such an output is shown in FIG. 12(b). According to FIG. 12(b), here, the first questions are output one by one, and the user answers one by one.

Thereafter, when the user has finished answering the fifteen first questions, the terminal processing unit 33 forms a group of first answers to be transmitted. The terminal transmission unit 34 transmits the formed group of first answers and the user identifier "U001" to the blood sugar constitution determination device 2.

Next, the first answer acceptance unit 221 of the blood sugar constitution determination device 2 receives the group of one or more first answers and the user identifier "U001".

Next, the first judgment unit 231 forms a first vector that is a vector whose elements are the first answers, using the received group of one or more first answers. That is to say, the first judgment unit 231 acquires the user attribute values paired with the user identifier "U001" (age, weight, BMI, and height in this example) from the user information management table shown in FIG. 9. Also, the first judgment unit 231 acquires the received group of fifteen first answers. Thereafter, the first judgment unit 231 forms a first vector whose elements are the user attribute values and the group of fifteen first answers (for example, (56, 78, 27.0, 170, first answer 1, first answer 2, ..., first answer 15)).

Next, the first judgment unit 231 acquires the first learner from the source information storage unit 213. Thereafter, the first judgment unit 231 acquires risk presence/absence information by performing first prediction processing, which is machine learning prediction processing, using the first vector and the acquired first learner. Here, it is assumed that the first judgment unit 231 has acquired risk presence/absence information indicating that "there is a risk "1"".

Next, the processing unit 23 acquires screen information containing a second questionnaire corresponding to "there is a risk "1"", from the second question storage unit 212.

Next, the second question output unit 242 outputs screen information containing the second questionnaire to the user terminal 3.

Next, the terminal reception unit 35 receives information from the blood sugar constitution determination device 2. Next, the terminal output unit 36 outputs the received information. An example of such an output is shown in FIG. 12(c).

Next, it is assumed that the user A has inputted an instruction corresponding to "NEXT, YOUR BLOOD SUGAR CONSTITUTION TYPE WILL BE DETERMINED" in response to FIG. 12(c). Accordingly, the user terminal 3 outputs the second questions contained in the second questionnaire ash shown in FIG. 12(d).

Next, it is assumed that the user answers the eight second questions contained in the second questionnaire one by one. Thereafter, the terminal acceptance unit 32 accepts a group of second answers from the user.

Next, the terminal processing unit 33 forms a group of second answers to be transmitted. The terminal transmission unit 34 transmits the formed group of second answers to the blood sugar constitution determination device 2.

Next, the second answer acceptance unit 222 of the blood sugar constitution determination device 2 receives a group of eight second answers.

Next, the second judgment unit 232 acquires the user attribute values paired with the user identifier "U001" (age, weight, BMI, and height in this example) from the user information management table shown in FIG. 9. Also, the second judgment unit 232 acquires the received group of eight second answers. Thereafter, the second judgment unit 232 forms a second vector whose elements are the user attribute values, the acquired group of one or more first answers, and the group of eight second answers (for example, (56, 78, 27.0, 170, first answer 1, first answer 2, ..., second answer 1, second answer 2, ..., second answer 8)).

Next, the second judgment unit 232 acquires the second learner from the source information storage unit 213. Next, it is assumed that the second judgment unit 232 acquires the estimated type "2" by performing second prediction processing, which is machine learning prediction processing, using the acquired second vector and the acquired second learner.

Next, the output information formation unit 233 acquires advice information paired with the type "2" from the advice storage unit 214. Such advice information is, for example, information on the screen shown in FIG. 12(f).

Next, the output information formation unit 233 forms output information, using the acquired estimated type "2" and the acquired advice information.

Next, the information output unit 243 transmits the output information to the user terminal 3.

Next, the terminal reception unit 35 of the user terminal 3 receives the output information from the blood sugar constitution determination device 2. Next, the terminal output unit 36 outputs the received output information. An example of such an output is shown in FIG. 12(e)(f).

As described above, according to the present embodiment, each user can easily know the blood sugar constitution type of the user by answering simple questions.

In addition, according to the present embodiment, by providing questions to users in two stages, it is possible for users with no blood sugar risk to determine that there is no risk, using answers to a small number of questions, which reduces the burden on the users.

In addition, according to the present embodiment, the blood sugar constitution type of each user is determined in the second step using the first answers as well, and therefore a user who has a risk related to blood sugar can determine the blood sugar constitution type, using answers to a small number of questions, which reduces the burden on the users.

Furthermore, according to the present embodiment, it is possible to provide advice corresponding to the user's blood sugar constitution type.

Note that the processing in the present embodiment may be realized using software. This software may be distributed through software downloading or the like. Also, this software may be recorded on a recording medium such as a CD-ROM and distributed. Note that the same applies to the other embodiments in the present description. Note that the software that realizes the blood sugar constitution determination device 2 according to the present embodiment is the program described below. That is to say, this program is a program that enables a computer that can access a storage unit that stores one or more pieces of question information that are used to judge a blood sugar constitution type and include a question regarding lifestyle, to function as: an output unit that outputs the one or more pieces of question information; an acceptance unit that accepts, from a user, answer information corresponding to the one or more pieces of question information; a processing unit that determines a blood sugar constitution type of the user, using the one or more pieces of answer information accepted by the acceptance unit; and an information output unit that outputs output information regarding the blood sugar constitution type.

### Second Embodiment

The present embodiment describes the simple questionnaire acquisition device B that acquires the questionnaire, the first questionnaire, or the second questionnaire mentioned in the first embodiment.

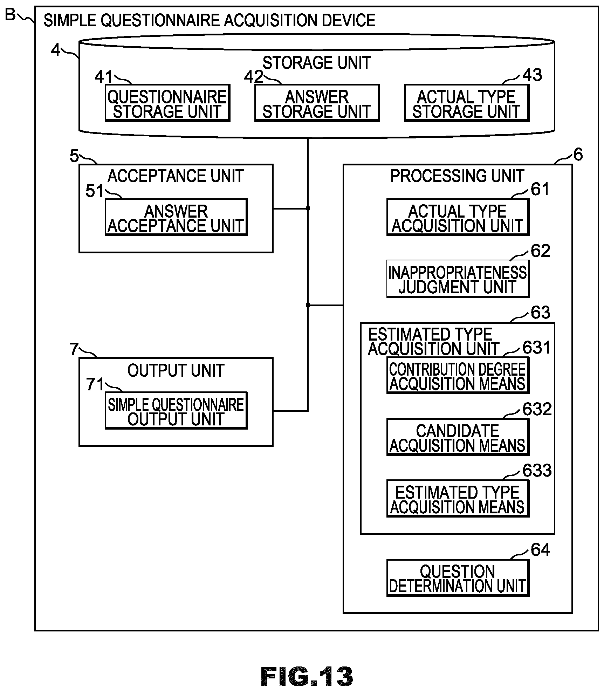

FIG. 13 is a block diagram for the simple questionnaire acquisition device B according to the present embodiment. The simple questionnaire acquisition device B includes a storage unit 4, an acceptance unit 5, a processing unit 6, and an output unit 7. The storage unit 4 includes a questionnaire storage unit 41, an answer storage unit 42, and an actual type storage unit 43. The acceptance unit 5 includes an answer acceptance unit 51. The processing unit 6 includes an actual type acquisition unit 61, an inappropriateness judgment unit 62, an estimated type acquisition unit 63, and a question determination unit 64. The estimated type acquisition unit 63 includes a contribution degree acquisition means 631, a candidate acquisition means 632, and an estimated type acquisition means 633. The output unit 7 includes a simple questionnaire output unit 71.

The storage unit 4 stores various kinds of information. Examples of the various kinds of information include a questionnaire, which will be described later, answers, which will be described later, actual types, which will be described later, an inappropriateness condition, which will be described later, and an approximation condition, which will be described later.

The questionnaire storage unit 41 stores a questionnaire. The questionnaire is a group of N questions (N is a natural number). The questionnaire is, for example, a group of questions including M₁ questions (M₁ is a natural number and M₁ < N) that are used to acquire an estimated type. The questionnaire is, for example, a group of questions including M₂ questions (M₂ is a natural number and M₂ < N) that are used to acquire estimated risk presence/absence information.

The questionnaire is, for example, a group of questions that are used to estimate the blood sugar constitution type of a user. The questionnaire is, for example, a group of questions that are used to determine whether or not a user has a risk related to blood sugar. However, there is no limitation on the content of the questionnaire, the number of questions, and so on. Note that the questions may be referred to as a query, an inquiry, or the like. The questionnaire may also be referred to as a survey. The questionnaire contains, for example, 350 questions.

The answer storage unit 42 stores answers. The answer storage unit 42 stores answers associated with identifiers. The answers are the user's answers to the questions contained in the questionnaire. Note that users are respondents. The answers are associated with the question identifiers of the questions contained in the questionnaire. The question identifiers are information identifying the questions, such as a question numbers and question IDs.

The identifiers are pieces of information identifying a group of answers, and are, for example, user identifiers, day identifiers, or user identifiers and day identifiers. User identifiers are information that identifies users, and are for example, IDs, e-mail addresses, telephone numbers, or the IDs of terminals used by the users (for example, IP addresses, MAC addresses, terminal identifiers, or the like).

Answers may be associated with user attribute values. User attribute values are the attribute values of each user, such as the sex, the age, and the occupation.

The actual type storage unit 43 stores two or more actual types. The actual types are typically associated with identifiers.

It is preferable that the actual types are associated with the answers. The actual types may be associated with the usage purposes of the actual types. That is to say, the actual type storage unit 43 may store actual types for learning and actual types for testing separately. The actual type storage unit 43 may store actual types for learning, actual types for verification, and actual types for testing separately.

The acceptance unit 5 accepts various kinds of instructions and information. Examples of the various kinds of instructions and information include answers and a simple questionnaire output instruction. The simple questionnaire output instruction is an instruction to output a simple questionnaire.

Any input means, such as a touch panel, a keyboard, a mouse, a menu screen, or the like, may be employed to input various kinds of information and instructions.

The answer acceptance unit 51 accepts answers. The answers are answers to the questions contained in the questionnaire. The answers are typically associated with user identifiers.

For example, the answer acceptance unit 51 accepts a group of answers to the questions contained in the questionnaire, and accumulates the group of answers in the answer storage unit 42 in association with user identifiers.

The processing unit 6 performs various kinds of processing. Examples of the various kinds of processing include the processing that is performed by the actual type acquisition unit 61, the inappropriateness judgment unit 62, the estimated type acquisition unit 63, the question determination unit 64, the contribution degree acquisition means 631, the candidate acquisition means 632, and the estimated type acquisition means 633.

The actual type acquisition unit 61 acquires actual type identifiers from the actual type storage unit 43. The actual type acquisition unit 61 acquires actual types paired with the group of answers to be processed, from the actual type storage unit 43. An actual type paired with the group of answers to be processed is, for example, the actual types associated with the same identifier as the group of answers.

The inappropriateness judgment unit 62 judges whether or not the answers to the N questions satisfy a predetermined inappropriateness condition. For example, the inappropriateness condition is that answers to the same two or more questions are different. For example, the inappropriateness condition is that a percentage no less than a threshold value (for example, no less than 95%) of the answers or a number no less than a threshold value of answers are the same answers (for example, all the answers are the same). It is preferable that the estimated type acquisition unit 63 and the question determination unit 64 do not use the group of answers judged by the inappropriateness judgment unit 62 as satisfying the inappropriateness condition.

The estimated type acquisition unit 63 acquires an estimated type, using answers to M₁ questions of N questions, where M₁ is less than N. Note that N is a natural number no less than 2. M₁ is a natural number no less than 1.

The estimated type acquisition unit 63 acquires estimated risk presence/absence information, using answers to M₂ questions of N questions, where M₂ is less than N. Note that N is a natural number no less than 2. M₂ is a natural number no less than 1.

The estimated type acquisition unit 63 acquires an estimated type identifier, using the contribution degree acquisition means 631, the candidate acquisition means 632, and the estimated type acquisition means 633. The estimated type acquisition unit 63 may acquire estimated risk presence/absence information, using the contribution degree acquisition means 631, the candidate acquisition means 632, and the estimated type acquisition means 633.

The contribution degree acquisition means 631 acquires the degree of contribution of answers to the N questions with respect to the actual type. Note that the degree of contribution of answers to questions may also be referred to as the degree of contribution of the questions. The contribution degree acquisition means 631 acquires the degree of contribution of the questions using the method (1) or (2) described below, for example. However, there is no limitation on the method for acquiring the degree of contribution of the questions.

The contribution degree acquisition means 631 acquires the degree of contribution of answers to the N answers with respect to the actual risk presence/absence information, for example. Note that the degree of contribution of answers to the questions may also be referred to as the degree of contribution of the questions. The contribution degree acquisition means 631 acquires the degree of contribution of the questions using the method (1) or (2) described below, for example. However, there is no limitation on the method for acquiring the degree of contribution of the questions.

### (1) In the case of acquiring the degree of contribution based on a random forest learner

### (1-1) In the case of acquiring the degree of contribution of questions for acquiring an estimated type

The contribution degree acquisition means 631 acquires the degree of contribution of answers, using a random forest module.

For example, the contribution degree acquisition means 631 acquires answers to the questions contained in a questionnaire containing N questions. For example, the contribution degree acquisition means 631 acquires, for each identifier, an actual type paired with the identifier, from the actual type storage unit 43. Next, the contribution degree acquisition means 631 forms, for each identifier, a vector whose elements are the answers and the actual type. Next, for example, the contribution degree acquisition means 631 provides vectors corresponding to two or more identifiers to the random forest learning module, and executes the random forest learning module to acquire a learner. Thereafter, the contribution degree acquisition means 631 acquires the degree of contribution of the questions corresponding to the answers, using the learner. The degree of contribution of the questions is information related to the contribution of the questions for acquiring the actual type, and may be referred to as a contribution rate, the degree of importance, or the like. The processing performed to acquire the degree of contribution of the questions corresponding to the answers, using the learner acquired by executing a random forest learning module is a well-known technique.

### (1-2) In the case of acquiring the degree of contribution of questions for acquiring estimated risk presence/absence information

The contribution degree acquisition means 631 acquires the degree of contribution of answers, using a random forest module.

For example, the contribution degree acquisition means 631 acquires answers to the questions contained in a questionnaire containing N questions. For example, the contribution degree acquisition means 631 acquires, for each identifier, actual risk presence/absence information paired with the identifier, from the actual type storage unit 43. Next, the contribution degree acquisition means 631 forms, for each identifier, a vector whose elements are the answers and the actual risk presence/absence information. Next, for example, the contribution degree acquisition means 631 provides vectors corresponding to two or more identifiers to the random forest learning module, and executes the random forest learning module to acquire a learner. Thereafter, the contribution degree acquisition means 631 acquires the degree of contribution of the questions corresponding to the answers, using the learner. The degree of contribution of the questions is information related to the contribution of the questions for acquiring the actual risk presence/absence information, and may be referred to as a contribution rate, the degree of importance, or the like. The processing performed to acquire the degree of contribution of the questions corresponding to the answers, using the learner acquired by executing a random forest learning module is a well-known technique.

### (2) In the case of acquiring the degree of contribution, using the result of an evaluation of the accuracy of a machine learning learner.

### (2-1) In the case of acquiring the degree of contribution of questions for acquiring an estimated type

(1-1) As in the processing described in (1-1), for example, the contribution degree acquisition means 631 forms, for each identifier, a vector whose elements are the answers to all the questions and the actual type. At this time, the contribution degree acquisition means 631 has acquired two or more vector. Next, the contribution degree acquisition means 631 acquires the basic accuracy, which is the accuracy (any of the accuracies such as the correct answer rate, the precision rate, the recall rate, and the F value) of the learner formed using the answers to all the questions and the actual type, by performing k-fold cross-validation, for example.

Next, for example, the contribution degree acquisition means 631 forms, for each identifier, a vector whose elements are answers excluding the answers to one question of all the questions, and the actual type. Next, the contribution degree acquisition means 631 acquires missing accuracy, which is the accuracy of the learner formed using the answers excluding the answers to one question and the actual type by k-fold cross-validation, for example. Next, the contribution degree acquisition means 631 calculates the difference between the basic accuracy and the missing accuracy. Next, for example, the contribution degree acquisition means 631 calculates the degree of contribution of the questions to the excluded answers, using an increasing function with this difference as a parameter. Note that the degree of contribution of the questions to the excluded answers can be calculated by the arithmetic expression "f(basic accuracy - missing accuracy)". The greater the difference between the basic accuracy and the missing accuracy is, the greater the degree of contribution of the excluded answers, i.e., the excluded questions, is. To exclude answers means to exclude the questions corresponding to the answers.

Thereafter, for example, the contribution degree acquisition means 631 makes changes to the first to N^{th} questions of the excluded questions, and acquires N missing accuracies for each of the excluded questions by performing k-fold cross-validation.

Note that the contribution degree acquisition means 631 uses a machine learning module to perform k-fold cross-validation. The machine learning module may be a random forest, a decision tree, deep learning, an SVR, or the like, and there is no limitation on the algorithm thereof. In addition, for machine learning, it is possible to use various machine learning functions in the TensorFlow library, fastText, tinySVM, an R language random forest module, or the like, or various existing libraries, for example. The k-fold cross-validation is a well-known technique, and therefore the detailed description thereof will be omitted.

In addition, in the method described in (2-1), the contribution degree acquisition means 631 may use only the N missing accuracies without using the basic accuracy to acquire the contribution degree of the questions, for example. That is to say, the contribution degree acquisition means 631 may acquire the contribution degree so that the lower the accuracy indicated by the missing accuracy corresponding to the missing questions is, the greater the contribution of the questions is. For example, the contribution degree acquisition means 631 may use a decreasing function having the missing accuracy of the questions as a parameter, to acquire the contribution degree of the questions.

### (2-2) In the case of acquiring the degree of contribution of questions for acquiring estimated risk presence/absence information

As in the processing described in (1-2), for example, the contribution degree acquisition means 631 forms, for each identifier, a vector whose elements are the answers to all the questions and the actual risk presence/absence information. At this time, the contribution degree acquisition means 631 has acquired two or more vector. Next, the contribution degree acquisition means 631 acquires the basic accuracy, which is the accuracy (any of the accuracies such as the accuracy rate, the precision rate, the recall rate, and the F value) of the learner formed using the answers to all the questions and the actual risk presence/absence information, by performing k-fold cross-validation, for example.

Next, for example, the contribution degree acquisition means 631 forms, for each identifier, a vector whose elements are answers excluding the answers to one question of all the questions, and the actual risk presence/absence information. Next, the contribution degree acquisition means 631 acquires missing accuracy, which is the accuracy of the learner formed using the answers excluding the answers to one question and the actual risk presence/absence information by k-fold cross-validation, for example. Next, the contribution degree acquisition means 631 calculates the difference between the basic accuracy and the missing accuracy. Next, for example, the contribution degree acquisition means 631 calculates the degree of contribution of the questions to the excluded answers, using an increasing function with this difference as a parameter. Note that the degree of contribution of the questions to the excluded answers can be calculated by the arithmetic expression "f(basic accuracy - missing accuracy)". The greater the difference between the basic accuracy and the missing accuracy is, the greater the degree of contribution of the excluded answers, i.e., the excluded questions, is. To exclude answers means to exclude the questions corresponding to the answers.

Thereafter, for example, the contribution degree acquisition means 631 makes changes to the first to N^{th} questions of the excluded questions, and acquires N missing accuracies for each of the excluded questions by performing k-fold cross-validation.

Note that the contribution degree acquisition means 631 uses a machine learning module to perform k-fold cross-validation as described above.

In addition, in the method described in (2-2), the contribution degree acquisition means 631 may use only the N missing accuracies without using the basic accuracy to acquire the contribution degree of the questions, for example. That is to say, the contribution degree acquisition means 631 may acquire the contribution degree so that the lower the accuracy indicated by the missing accuracy corresponding to the missing questions is, the greater the contribution of the questions is. For example, the contribution degree acquisition means 631 may use a decreasing function having the missing accuracy of the questions as a parameter, to acquire the contribution degree of the questions.

The candidate acquisition means 632 acquires two or more question group candidates. A question group candidate is a candidate for a question group. Question groups are two or more groups of two or more different number of questions, of the N questions contained in a questionnaire. There is no limitation on the method by which the candidate acquisition means 632 acquires two or more question group candidates. The candidate acquisition means 632 acquires two or more question group candidates using any of the methods (1) to (3) described below, for example.

### (1) In the case of using a degree of contribution

The candidate acquisition means 632 acquires two or more question group candidates, which are groups of questions whose contribution degrees acquired by the contribution degree acquisition means 631 satisfy a predetermined contribution degree condition, for example. Note that the contribution degree condition is that the degree of contribution is large. The contribution degree condition is, for example, that the degree of contribution is no less than a threshold value, or is greater than the threshold value, or is in the top N or higher.

The candidate acquisition means 632 acquires two or more question group candidates that contain a greater number of questions for a higher degree of contribution, for example. The two or more question group candidates that contain a greater number of questions for a higher degree of contribution are, for example, "a question with the highest degree of contribution", "a group of two questions with the 1^{st} and 2^{nd} highest degrees of contribution", "a group of three questions with the 1^{st} to 3^{rd} highest degrees of contribution", ..., and "a group of X questions with the 1^{st} to X^{th} highest degrees of contribution". Note that there is no limitation on the value of X.

### (2) In the case of acquiring all the combinations

For example, the candidate acquisition means 632 acquires, from a questionnaire that is a group of N questions, N question group candidates each consisting of one question, _{N}C₂ question group candidates, _{N}C₃ question group candidates, _{N}C₄ question group candidates, _{N}C_{N-1} question group candidates, and one question group candidate consisting of N questions. That is to say, the candidate acquisition means 632 acquires (_{N}C₁ + _{N}C₂ + _{N}C₃ + ... + _{N}C_{N-2} + _{N}C_{N-1} + _{N}C_{N}) question group candidates from the questionnaire that is a group of N questions. In such a case, the contribution degree acquisition means 631 is unnecessary. Here, the upper limit may be set for the number of questions contained in a group. For example, the candidate acquisition means 632 may be configured not to acquire a group of N questions.

### (3) In the case of random acquisition

For example, the candidate acquisition means 632 randomly selects a first question group candidate consisting of two questions, to acquire X first question group candidates (X is determined in advance, for example). Also, for example, the candidate acquisition means 632 randomly selects a second question group candidate consisting of three questions, to acquire X second question group candidates. Also, for example, the candidate acquisition means 632 randomly selects a third question group candidate consisting of four questions, to acquire X third question group candidates. In this way, the candidate acquisition means 632 randomly selects a question group candidate consisting of Y questions (Y < N, Y is determined in advance), to acquire X question group candidates. Even in such a case, the contribution degree acquisition means 631 is unnecessary.

For each of the two or more question group candidates acquired by the candidate acquisition means 632, the estimated type acquisition means 633 acquires an estimated type that is a value acquired using the answers to the two or more questions contained in the question group candidate. The estimated type acquisition means 633 acquires the estimated type, using the method (1) or (2) described below, for example.

### (1) A method in which a learner is formed using answers and actual types for learning, and an estimated type identifier is acquired using the learner and answers for testing

For each of the two or more question group candidates acquired by the candidate acquisition means 632, and for each identifier, the estimated type acquisition means 633 acquires answers for learning corresponding to the two or more questions contained in the question group candidate, from the answer storage unit 42. Also, for each of the two or more question group candidates acquired by the candidate acquisition means 632, and for each user identifier, the estimated type acquisition means 633 acquires an actual type corresponding to the answers for learning, from the actual type storage unit 43.

Next, for each of the two or more question group candidates acquired by the candidate acquisition means 632, the estimated type acquisition means 633 provides answers and actual types paired with the two or more identifiers to a machine learning module, and performs learning processing to acquire a learner. Although random forest is suitable for machine learning, another algorithm such as deep learning, a decision tree, or an SVR, or the like may be used.

Next, for each of the two or more question group candidates acquired by the candidate acquisition means 632, and for each identifier, the estimated type acquisition means 633 acquires answers for testing corresponding to the two or more questions contained in the question group candidate, from the answer storage unit 42. Thereafter, for each of the two or more question group candidates and for each identifier, the estimated type acquisition means 633 provides the acquired learner and the answers for testing to a machine learning prediction module, and executes the prediction module to acquire an estimated type. Note that the estimated type may also be referred to as a predicted type or the like.

### (2) A method using k-fold cross-validation

For each of the two or more question group candidates acquired by the candidate acquisition means 632, and for each identifier, the estimated type acquisition means 633 acquires answers to the two or more questions contained in the question group candidate, from the answer storage unit 42. Also, for each of the two or more question group candidates acquired by the candidate acquisition means 632, the estimated type acquisition means 633 acquires an actual type paired with the identifier, from the actual type storage unit 43.

Next, for each of the two or more question group candidates, and for each identifier, the estimated type acquisition means 633 acquires an estimated type by performing k-fold cross-validation. That is to say, for each of the two or more question group candidates, for example, the group of answers of all the users and the actual types are divided into k sets (for example, 10 sets), and (k-1)/k of the data are provided to the machine learning module, and the learning module is executed to acquire a learner. Next, the estimated type acquisition means 633 provides the remaining 1/k of data and the learner to the machine learning prediction module, and executes the prediction module to acquire the group of answers corresponding to the remaining 1/k of the data and the estimated type. Thereafter, the estimated type acquisition means 633 executes the above-described processing k times while changing the data to be provided to the learning module and the data to be provided to the prediction module, thereby acquiring an estimated type for each identifier, for each of the two or more question group candidates.

Alternatively, for each of the two or more question group candidates acquired by the candidate acquisition means 632, the estimated type acquisition means 633 may acquire estimated risk presence/absence information that can be acquired using answers to the two or more questions contained in the question group candidate. As in the case of acquiring the estimated type, the estimated type acquisition means 633 acquires the estimated risk presence/absence information, using the method (1) or (2) described below, for example.

### (1) A method in which a learner is formed using answers actual risk presence/absence information for learning, and an estimated risk presence/absence information is acquired using the learner and answers for testing

For each of the two or more question group candidates acquired by the candidate acquisition means 632, and for each identifier, the estimated type acquisition means 633 acquires answers for learning corresponding to the two or more questions contained in the question group candidate, from the answer storage unit 42. Also, for each of the two or more question group candidates acquired by the candidate acquisition means 632, and for each user identifier, the estimated type acquisition means 633 acquires actual risk presence/absence information corresponding to the answers for learning, from the actual type storage unit 43.

Next, for each of the two or more question group candidates acquired by the candidate acquisition means 632, the estimated type acquisition means 633 provides answers and actual risk presence/absence information paired with the two or more identifiers to a machine learning module, and performs learning processing to acquire a learner.

Next, for each of the two or more question group candidates acquired by the candidate acquisition means 632, and for each identifier, the estimated type acquisition means 633 acquires answers for testing corresponding to the two or more questions contained in the question group candidate, from the answer storage unit 42. Thereafter, for each of the two or more question group candidates and for each identifier, the estimated type acquisition means 633 provides the acquired learner and the answers for testing to a machine learning prediction module, and executes the prediction module to acquire estimated risk presence/absence information.

### (2) A method using k-fold cross-validation

For each of the two or more question group candidates acquired by the candidate acquisition means 632, and for each identifier, the estimated type acquisition means 633 acquires answers to the two or more questions contained in the question group candidate, from the answer storage unit 42. Also, for each of the two or more question group candidates acquired by the candidate acquisition means 632, the estimated type acquisition means 633 acquires actual risk presence/absence information paired with the identifier, from the actual type storage unit 43.

Next, for each of the two or more question group candidates, and for each identifier, the estimated type acquisition means 633 acquires estimated risk presence/absence information by performing k-fold cross-validation. That is to say, for each of the two or more question group candidates, for example, the group of answers of all the users and the pieces of actual risk presence/absence information are divided into k sets (for example, 10 sets), and (k-1)/k of the data are provided to the machine learning module, and the learning module is executed to acquire a learner. Next, the estimated type acquisition means 633 provides the remaining 1/k of data and the learner to the machine learning prediction module, and executes the prediction module to acquire the group of answers corresponding to the remaining 1/k of the data and the estimated risk presence/absence information. Thereafter, the estimated type acquisition means 633 executes the above-described processing k times while changing the data to be provided to the learning module and the data to be provided to the prediction module, thereby acquiring risk presence/absence information for each identifier, for each of the two or more question group candidates.

When the accuracy regarding the degree of matching between the estimated type acquired by the estimated type acquisition unit 63 and the actual type satisfies a predetermined accuracy condition, the question determination unit 64 selects M questions corresponding to the estimated type. The group of such M questions is a questionnaire.

When the accuracy of the degree of matching between the estimated risk presence/absence information acquired by the estimated type acquisition unit 63 and the actual risk presence/absence information satisfies a predetermined accuracy condition, the question determination unit 64 selects M₁ questions corresponding to the estimated risk presence/absence information. The group of such M₁ questions is a first questionnaire.

When the accuracy of the degree of matching between the estimation type acquired by the estimated type acquisition unit 63 and the actual type satisfies a predetermined accuracy condition, the question determination unit 64 selects questions, which are M₂ questions corresponding to the estimation type. The group of such M₂ questions is a second questionnaire.

The output unit 7 outputs various kinds of information. Examples of the various kinds of information include a simple questionnaire, which will be described later.

Here, "output" is a concept that encompasses displaying on a display screen, projection using a projector, printing by a printer, the output of a sound, transmission to an external device, accumulation on a recording medium, delivery of a processing result to another processing device or another program, and the like.

The simple questionnaire output unit 71 outputs a simple questionnaire that is information regarding a question group, which is the group of M questions determined by the question determination unit 64. The simple questionnaire may be a group of M questions, or a group of the IDS of M questions, or a group of pieces of information regarding links to M questions. The simple questionnaire need only be information based on which the group of M questions can be acquired. In addition, the simple questionnaire to be output may be questions in which at least one of the M questions determined by the question determination unit 64 has been modified. Note that the simple questionnaire may be the first questionnaire or the second questionnaire described above.

It is preferable that the storage unit 4, the questionnaire storage unit 41, the answer storage unit 42, and the actual type storage unit 43 are realized using a non-volatile recording medium, but they may be realized using a volatile recording medium.

There is no limitation on the process in which information is stored in the storage unit 4 and so on. For example, information may be stored in the storage unit 4 or the like via a recording medium, or information transmitted via a communication line or the like may be stored in the storage unit 4 or the like, or information input via an input device may be stored in the storage unit 4 or the like.

The acceptance unit 5 and the answer acceptance unit 51 can be realized using a device driver for the input means such as a touch panel or a keyboard, or control software or the like for controlling the menu screen. Note that the acceptance unit 5 and so on may be realized using a wireless or wired communication means.

The processing unit 6, the actual type acquisition unit 61, the inappropriateness judgment unit 62, the estimated type acquisition unit 63, the question determination unit 64, the contribution degree acquisition means 631, the candidate acquisition means 632, and the estimated type acquisition means 633 can typically be realized using a processor, a memory, and so on. The processing procedures performed by the processing unit 6 and so on are typically realized using software, and the software is recorded on a recording medium such as a ROM. However, such processing procedures may be realized using hardware (a dedicated circuit). The processor is, for example, a CPU, an MPU, a GPU, or the like, and there is no limitation on the type thereof.

The output unit 7 and the simple questionnaire output unit 71 may be regarded as including or not including an output device such as a display or a speaker. The output unit 7 and so on can be realized using the driver software of the output device, the driver software of the output device and the output device, or the like. Note that the output unit 7 and so on may be realized using a wireless or wired communication means. In addition, the output unit 7 and so on may be realized using a processor, a memory, and so on.

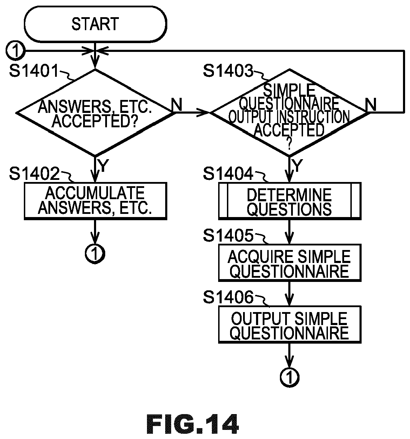

Next, an operation example of the simple questionnaire acquisition device B will be described with reference to the flowchart in FIG. 14.

(Step S1401) The answer acceptance unit 51 judges whether or not answers to the N questions contained in the questionnaire and the actual measurement value have been accepted, in pair with identifiers. Note that the actual measurement value is an actual type or risk presence/absence information. If answers and so on have been accepted, processing proceeds to step S1402, and if answers and so on have not been accepted, processing proceeds to step S1403. Note that the identifiers are user identifiers, for example. Alternatively, the identifiers are user identifiers and day identifiers, for example.

(Step S1402) The processing unit 6 accumulates the group of answers and the actual measurement value accepted in step S201 in the answer storage unit 42 in pair with the identifiers. The processing returns to step S1401.

(Step S1403) The acceptance unit 5 judges whether or not a simple questionnaire output instruction has been accepted. If a simple questionnaire output instruction has been accepted, processing proceeds to step S1404, and if a simple questionnaire output instruction has not been accepted, processing returns to step S1401.

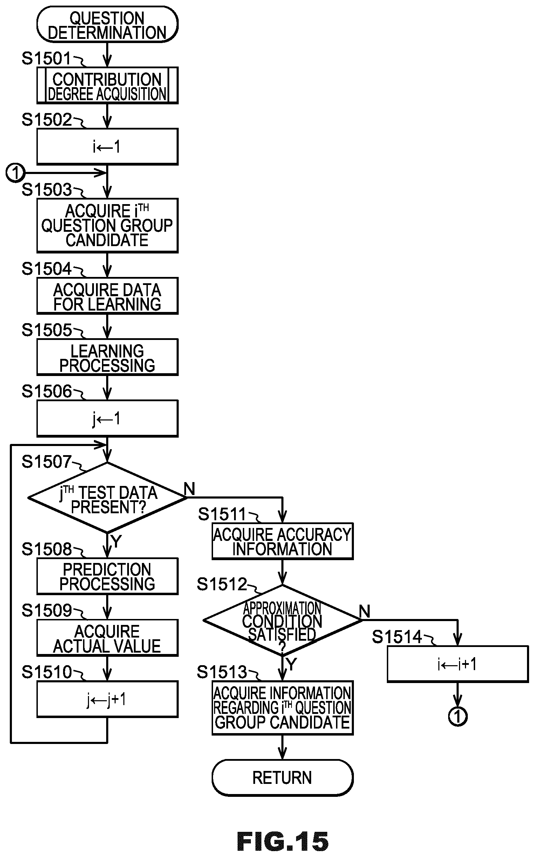

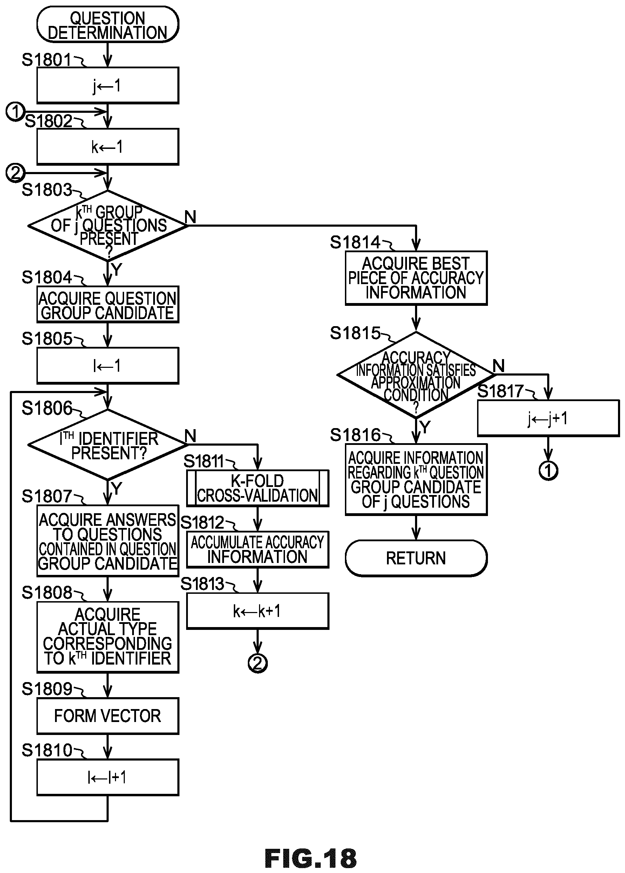

(Step S1404) The processing unit 6 performs question determination processing. An example of question determination processing will be described with reference to the flowcharts in FIGS. 15 and 18. Note that question determination processing is processing that is performed to determine questions for forming a simple questionnaire.

(Step S1405) The processing unit 6 acquires a simple questionnaire to be output, using the one or more questions determined in step S1404.

(Step S1406) The simple questionnaire output unit 71 outputs the simple questionnaire acquired in step 1407. The processing returns to step S1401. Here, for example, the simple questionnaire output unit 71 accumulates the simple questionnaire acquired in step S1407 in the questionnaire storage unit 41.

In the flowchart shown in FIG. 14, processing is terminated when power is turned off or an interruption is made to terminate the processing.

A first example of the question determination processing in step S1404 will be described with reference to the flowchart in FIG. 15.

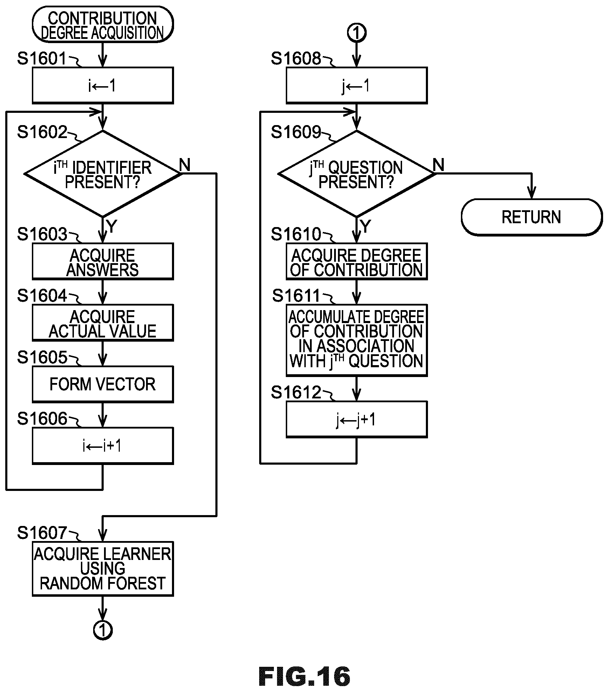

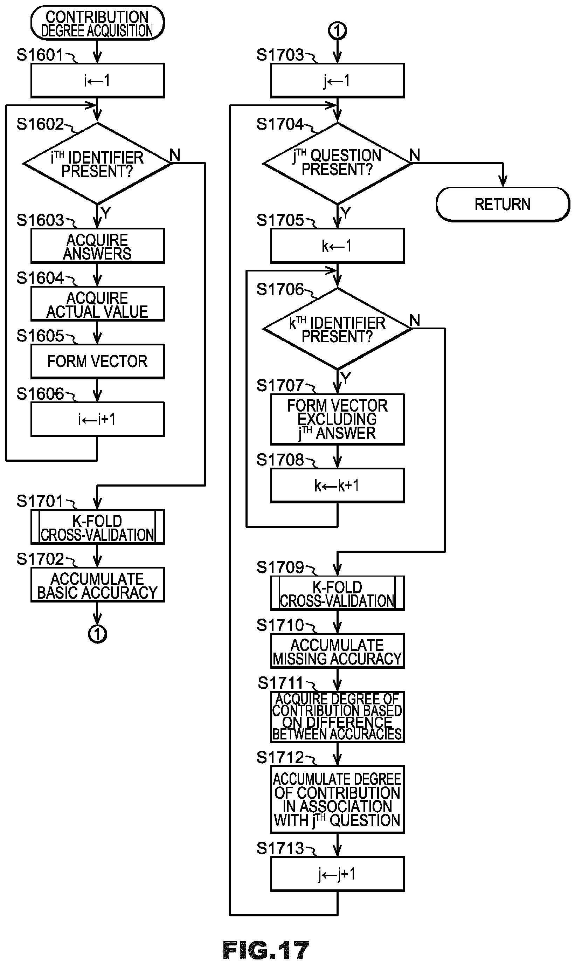

(Step S1501) The contribution degree acquisition means 631 acquires the degree of contribution of each of the questions in the questionnaire. An example of such contribution degree acquisition processing will be described with reference to the flowcharts in FIGS. 16 and 17.

(Step S1502) The estimated type acquisition unit 63 substitutes 1 for the counter i.

(Step S1503) The candidate acquisition means 632 acquires an i^{th} question group candidate, using the degree of contribution of each of the questions acquired in step S1501. For example, the candidate acquisition means 632 acquires i questions, namely the question with the highest degree of contribution to the question with the i^{th} highest degree of contribution, from the questionnaire in the questionnaire storage unit 41.

(Step S1504) The estimated type acquisition means 633 acquires data for learning from the answer storage unit 42.

That is to say, for example, the estimated type acquisition means 633 acquires a group of answers that is paired with the purpose "for learning", and is a group of answers to the questions contained in the i^{th} question group candidate, from the answer storage unit 42. Also, the estimated type acquisition means 633 acquires, for each identifier paired with the purpose "for learning", an actual measurement value paired with the identifier, from the actual type storage unit 43. Next, the estimated type acquisition means 633 acquires, for each identifier, a vector whose elements are answers to the questions and the actual measurement values. Note that such data for learning is a group of vectors corresponding to the two or more identifiers.

(Step S1505) The estimated type acquisition means 633 provides the data for learning acquired in step S1504 to a module for machine learning processing, and executes the module to acquire a learner. Note that the machine learning module is, for example, a random forest module.

(Step S1506) The estimated type acquisition means 633 substitutes 1 for the counter j.

(Step S1507) The estimated type acquisition means 633 judges whether or not a j^{th} piece of test data is stored in the answer storage unit 42. If a j^{th} piece of test data is present, processing proceeds to step S 1508, and if a j^{th} piece of test data is not present, processing proceeds to step S1511. Note that test data is a group of answers associated with identifiers.

(Step S1508) The estimated type acquisition means 633 acquires a group of answers that is the j^{th} piece of test data and is a group of answers containing, as elements, answers to the questions contained in the i^{th} question group candidate, from the pieces of test data stored in the answer storage unit 42. Next, the estimated type acquisition means 633 acquires such a group of answers and the learner acquired in step S1505 to a machine learning prediction module, and executes the prediction module to acquire an estimated value. Note that the estimated value is an estimated type or estimated risk presence/absence information.

(Step S1509) The question determination unit 64 acquires an identifier paired with the j^{th} piece of test data from the answer storage unit 42. Next, the question determination unit 64 acquires an actual measurement value paired with the identifier from the actual type storage unit 43, and associates the actual measurement value with the estimated value acquired in step S410.

(Step S1510) The estimated type acquisition means 633 increments the counter j by 1. The processing returns to step S1507.

(Step S1511) The question determination unit 64 acquires the same number of sets of the estimated value acquired in step S1508 and the actual measurement value acquired in step S409, as the number of pieces of test data. Next, using all the pieces of information of the sets of estimated values and actual measurement values, the question determination unit 64 acquires accuracy information regarding the accuracy of the estimated values with respect to the actual measurement values.

For example, using all the pieces of information of the sets of estimated values and the actual measurement values, the question determination unit 64 acquires accuracy information that is the rate of matching between estimated values and actual measurement values. For example, using all the pieces of information of the sets of estimated values and the actual measurement values, the question determination unit 64 acquires accuracy information that is a correlation coefficient between estimated values and actual measurement values.

(Step S1512) The question determination unit 64 judges whether or not the accuracy information acquired in step S413 satisfies the approximation condition stored in the storage unit 4. If the approximation condition is satisfied, processing proceeds to step S1513, and if the approximation condition is not satisfied, processing proceeds to step S1514. Note that the approximation condition is that accuracy information indicates a value no less than a threshold value, or that accuracy information indicates a value greater than a threshold value, for example.

(Step S1513) The question determination unit 64 acquires information regarding the i^{th} question group candidate. Processing returns to higher level processing. Note that information regarding the i^{th} question group candidate is, for example, the i^{th} question group candidate itself, or the question identifiers of the questions contained in the i^{th} question group candidate.

(Step S1514) The estimated type acquisition unit 63 increments the counter i by 1. The processing returns to step S1503.

Next, a first example of the contribution degree acquisition processing in step S1501 will be described with reference to the flowchart in FIG. 16.

(Step S1601) The contribution degree acquisition means 631 substitutes 1 for the counter i.

(Step S1602) The contribution degree acquisition means 631 judges whether or not the i^{th} identifier is present. If the i^{th} identifier is present, processing proceeds to step S1603, and if the i^{th} identifier is not present, processing proceeds to step S1607.

(Step S1603) The contribution degree acquisition means 631 acquires answers to all the questions corresponding to the i^{th} identifier.

(Step S1604) The contribution degree acquisition means 631 acquires an actual measurement value corresponding to the i^{th} identifier from the actual type storage unit 43. Note that the actual measurement value is an actual type or actual risk presence/absence information.

(Step S1605) The contribution degree acquisition means 631 forms a vector whose elements are all the answers acquired in step S1603 and the actual measurement value acquired in step S1604.

(Step S1606) The contribution degree acquisition means 631 increments the counter i by 1. The processing returns to step S1602.

(Step S1607) The contribution degree acquisition means 631 provides the all the vectors formed in step S1605 to a random forest learning module, and executes the learning module to acquire a learner. Note that the learner is a learner that can be used for random forest prediction processing, and is a learner to which the answers to all the questions corresponding to the type identifier of the type of interest are input and is used to output an estimated value.

(Step S1608) The contribution degree acquisition means 631 substitutes 1 for the counter j.

(Step S1609) The contribution degree acquisition means 631 judges whether or not a j^{th} question is present. If a j^{th} question is present, processing proceeds to step S1610, and if a j^{th} question is not present, processing returns to higher level processing.

(Step S1610) The contribution degree acquisition means 631 acquires a degree of contribution of the j^{th} question (the j^{th} feature value), using the learner acquired in step S1607. Note that the processing performed to acquire the degree of contribution of the j^{th} feature value using a random forest learner is a well-known technique.

(Step S1611) The contribution degree acquisition means 631 accumulates the degree of contribution acquired in step S1610 in association with the j^{th} question.

(Step S1612) The contribution degree acquisition means 631 increments the counter j by 1. The processing returns to step S1609.

Next, a second example of the contribution degree acquisition processing in step S1501 will be described with reference to the flowchart in FIG. 17. In the flowchart in FIG. 17, the descriptions of the same steps as in the flowchart in FIG. 16 will be omitted.

(Step S1701) Using the group of vectors acquired in step S1605, the contribution degree acquisition means 631 performs k-fold cross-validation according to a machine learning algorithm to acquire a basic accuracy that is the accuracy in the case where answers to all the target questions are used.

(Step S1702) The contribution degree acquisition means 631 temporarily accumulates the basic accuracy acquired in step S1701.

(Step S1703) The contribution degree acquisition means 631 substitutes 1 for the counter j.

(Step S1704) The contribution degree acquisition means 631 judges whether or not a j^{th} question is present in all the target questions. If a j^{th} question is present, processing proceeds to step S1705, and if a j^{th} question is not present, processing returns to higher level processing.

(Step S1705) The contribution degree acquisition means 631 substitutes 1 for a counter k.

(Step S1706) The contribution degree acquisition means 631 judges whether or not the k^{th} identifier is present. If the k^{th} identifier is present, processing proceeds to step S1707, and if the k^{th} identifier is not present, processing proceeds to step S1709.

(Step S1707) The contribution degree acquisition means 631 acquires a vector that is acquired in step S1605 and is paired with the k^{th} identifier, and excludes the answer to the j^{th} question from the vector to form a vector. That is to say, the contribution degree acquisition means 631 forms a vector whose dimension is smaller by 1 than the vector acquired in step S1605.

(Step S1708) The contribution degree acquisition means 631 increments the counter k by 1. The processing returns to step S1706.

(Step S1709) Using the group of vectors acquired in step S1707, the contribution degree acquisition means 631 performs k-fold cross-validation according to a machine learning algorithm to acquire a missing accuracy that is the accuracy in the case of using the answers except for the answer to the j^{th} question.

(Step S1710) The contribution degree acquisition means 631 temporarily accumulates the missing accuracy acquired in step S1709 in association with the j^{th} question.

(Step S1711) The contribution degree acquisition means 631 acquires the difference between the missing accuracy temporarily accumulated in step S1702 and the basic accuracy temporarily accumulated in step S1710. The greater such a difference is, the greater the degree of contribution of the answer to the j^{th} question is. Therefore, here, such a difference is regarded as the degree of contribution.

(Step S1712) The contribution degree acquisition means 631 accumulates the degree of contribution acquired in step S1711 in association with the j^{th} question.

(Step S1713) The contribution degree acquisition means 631 increments the counter j by 1. The processing returns to step S1704.

Next a second example of the question determination processing in step S1604 will be described with reference to the flowchart in FIG. 18.

(Step S1801) The question determination unit 64 substitutes 1 for the counter j.

(Step S1802) The question determination unit 64 substitutes 1 for the counter k.

(Step S1803) The question determination unit 64 judges whether or not the k^{th} combination of j questions is present. If the k^{th} combination is present, processing proceeds to step S1804, and if the k^{th} combination is not present, processing proceeds to step S1814.

(Step S1804) The question determination unit 64 acquires the question group candidates that is the k^{th} combination of j questions.

(Step S1805) The question determination unit 64 substitutes 1 for a counter 1.

(Step S1806) The question determination unit 64 judges whether or not the l^{th} identifier is present. If the l^{th} identifier is present, processing proceeds to step S1807, and if the l^{th} identifier is not present, processing proceeds to step S1811.

(Step S1807) The question determination unit 64 acquires answers that correspond to the questions contained in the question group candidates acquired in step S1804 and are paired with the l^{th} identifier, from the answer storage unit 42.

(Step S1808) The question determination unit 64 acquires an actual value corresponding to the i^{th} type and paired with the l^{th} identifier from the actual type storage unit 43.

(Step S1809) The question determination unit 64 forms a vector whose elements are the answers to the questions acquired in step S1807 and the actual value acquired in step S1808.

(Step S1810) The counter 1 is incremented by 1. The processing returns to step S1806.

(Step S1811) Using the group of vectors acquired in step S1809, the question determination unit 64 performs k-fold cross-validation to acquire accuracy information specifying the accuracy of the learner formed using the group of the vectors acquired in step S1809.

(Step S1812) The question determination unit 64 accumulates the accuracy information acquired in step S1811 in the buffer (not shown) in association with the k^{th} question group candidate.

(Step S1813) The question determination unit 64 increments the counter k by 1. The processing returns to step S1803.

(Step S1814) The question determination unit 64 acquires the best piece of accuracy information in the case of using j questions, of the pieces of accuracy information accumulated in step S1812.

(Step S1815) The question determination unit 64 judges whether or not the accuracy information acquired in step S1814 satisfies the approximation condition stored in the storage unit 4. If the approximation condition is satisfied, processing proceeds to step S1816, and if the approximation condition is not satisfied, processing proceeds to step S1817.

(Step S1816) The question determination unit 64 acquires information regarding the k^{th} question group candidate of j questions. Processing proceeds to step S416. Note that the k^{th} question group candidate of j answers is the group of answers determined by the question determination unit 64, and is the group of answers to be used to form a simple questionnaire.

(Step S1817) The question determination unit 64 increments the counter j by 1. The processing returns to step S1802.

Hereinafter, a specific operation example of the simple questionnaire acquisition device B according to the present embodiment will be described.

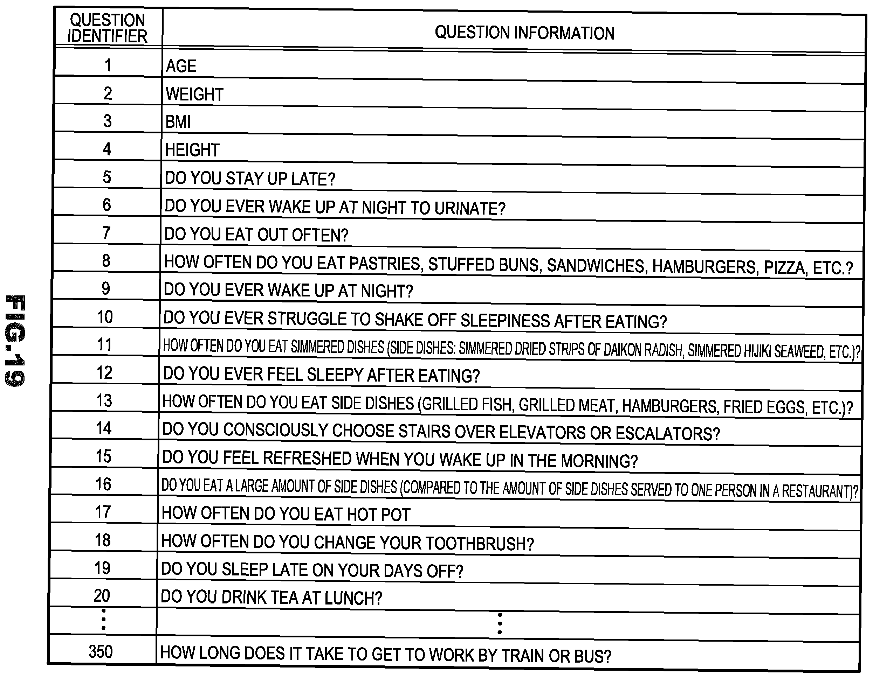

Now, the questionnaire shown in FIG. 19 is stored in the questionnaire storage unit 41. The questionnaire in this example contains 350 pieces of question information.

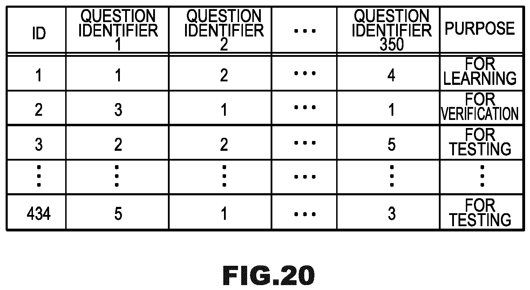

The answer storage unit 42 stores answers from 434 users to the questionnaire. Such answers are shown in FIG. 20. In FIG. 20, "ID" indicates the identifiers of the users who answered. In FIG. 20, the answers are associated with "Purpose". In this example, the purpose is "for learning", "for verification", or "for testing". The records corresponding to "for learning" are used to build a random forest learner. The records corresponding to "for verification" are used to adjust various parameters for the random forest learner. The records corresponding to "for testing" are used to acquire estimated values, acquire accuracy information between the estimated values and the actual measurement values, and determine the last question. Note that "purpose" may be automatically or manually added.

Furthermore, although not shown in the figure, the actual type storage unit 43 stores actual risk presence/absence information and actual types in association with the identifiers of the users.

In the above situation, first, the contribution degree acquisition means 631 in the simple questionnaire acquisition device B acquires the degree of contribution of each of the questions in the case of acquiring estimated risk presence/absence information according to the algorithm in FIG. 16 or 17 described above, and temporarily accumulates the degree of contribution in association with the questions.

Next, the candidate acquisition means 632 and the question determination unit 64 determine the questions to be contained in the simple questionnaire in the case of acquiring estimated risk presence/absence information according to the algorithm in FIG. 15. The questions determined here are, for example, the fifteen questions shown in FIG. 10.

Note that the question determination unit 64 and so on may determine the questions to be contained in the simple questionnaire in the case of acquiring estimated risk presence/absence information according to the algorithm in FIG. 18.

Next, the contribution degree acquisition means 631 in the simple questionnaire acquisition device B acquires the degree of contribution of each of the questions in the case of acquiring an estimated type according to the algorithm in FIG. 16 or 17 described above, and temporarily accumulates the degree of contribution in association with the questions.

Next, the candidate acquisition means 632 and the question determination unit 64 determine the questions to be contained in the simple questionnaire in the case of acquiring an estimated type according to the algorithm in FIG. 15. The questions determined here are, for example, the eight questions shown in FIG. 11.

Note that the question determination unit 64 and so on may determine the questions to be contained in the simple questionnaire in the case of acquiring an estimated type according to the algorithm in FIG. 18.

As described above, according to the present embodiment, it is possible to acquire a simple questionnaire with a smaller number of questions to estimate a risk type.

In addition, according to the present embodiment, it is possible to acquire a simple questionnaire with a smaller number of questions to estimate risk presence/absence information.

Furthermore, the processing in the present embodiment may be realized using software. This software may be distributed through software downloading or the like. Also, this software may be recorded on a recording medium such as a CD-ROM and distributed. Note that the same applies to the other embodiments in the present description. Note that the software that realizes the simple questionnaire acquisition device B according to the present embodiment is the program described below. That is to say, this program is a program that enables a computer to function as: an estimated type acquisition unit that acquires an estimated type identifier, using answers to M questions out of answers that are answers to N questions and that correspond to any actual type identifier of two or more actual type identifiers, where M is less than N; a question determination unit that, when an accuracy regarding a degree of matching between the estimated type identifier acquired by the estimated type acquisition unit and the actual type identifier satisfies a predetermined accuracy condition, determines M questions corresponding to the estimated type identifier; and a simple questionnaire output unit that outputs a simple questionnaire that is information regarding a question group that is a group of the M questions determined by the question determination unit.

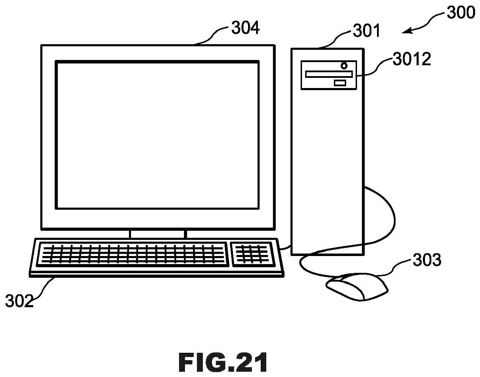

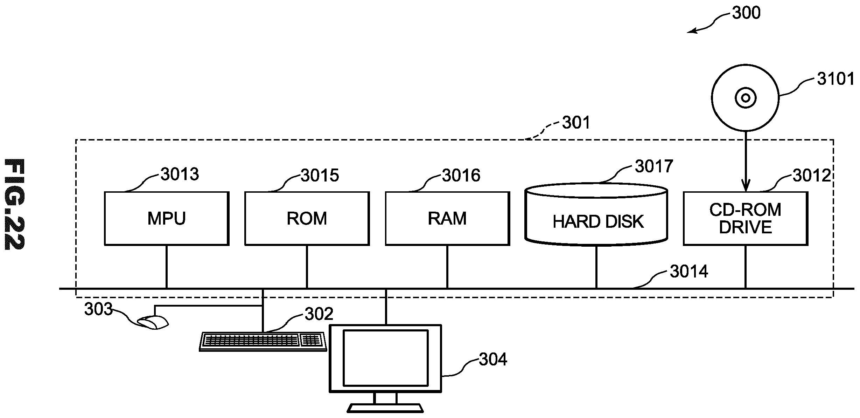

FIG. 21 shows the external appearance of a computer that executes the program described herein to realize the learning device 1, the blood sugar constitution determination device 2, the simple questionnaire acquisition device B, and so on according to the various embodiments described above. The above-described embodiments can be realized using computer hardware and a computer program that runs thereon. FIG. 21 is an overview diagram for this computer system 300 and FIG. 22 is a block diagram for the system 300.

In FIG. 21, the computer system 300 includes a computer 301 that includes a CD-ROM drive, a keyboard 302, a mouse 303, and a monitor 304.

In FIG. 22, the computer 301 includes, in addition to the CD-ROM drive 3012, an MPU 3013, a bus 3014 that is connected to the CD-ROM drive 3012 and so on, a ROM 3015 for storing programs such as a boot-up program, a RAM 3016 that is connected to the MPU 3013 and is used to temporarily store application program instructions and provide a temporary storage space, and a hard disk 3017 for storing application programs, system programs, and data. Here, although not shown in the drawings, the computer 301 may further include a network card that provides connection to a LAN.

The program that enables the computer system 300 to perform the functions of the blood sugar constitution determination device 2 and so on according to the above-described embodiments may be stored in the CD-ROM 3101, inserted into the CD-ROM drive 3012, and furthermore transferred to the hard disk 3017. Alternatively, the program may be transmitted to the computer 301 via a network (not shown) and stored on the hard disk 3017. The program is loaded into the RAM 3016 when the program is to be executed. The program may be directly loaded from the CD-ROM 3101 or the network.

The program does not necessarily have to include an operating system (OS), a third party program, or the like that enables the computer 301 to perform the functions of the blood sugar constitution determination device 2 and so on according to the embodiments described above. The program need only contain the part of the instruction that calls an appropriate function (module) in a controlled manner to achieve a desired result. How the computer system 300 works is well known and the detailed descriptions thereof will be omitted.

In the above-described program, the step of transmitting information, the step of receiving information and so on do not include processing performed by hardware, for example, processing performed by a modem or an interface card in the step of transmitting (processing that can only be performed by hardware).

There may be a single or multiple computers executing the above-described program. That is to say, centralized processing or distributed processing may be performed.

Also, as a matter of course, in each of the above-described embodiments, two or more communication means that are present in one device may be physically realized using one medium.

In each of the above-described embodiments, each kind of processing may be realized as centralized processing that is performed by a single device, or distributed processing that is performed by multiple devices.

As a matter of course, the present invention is not limited to the above-described embodiments, and various changes are possible, and such variations are also included within the scope of the present invention.

### Industrial Applicability

As described above, a blood sugar constitution determination device according to the present invention achieves the effect that the user's blood sugar constitution type can be easily known, and is useful as a blood sugar constitution determination device or the like.

## Claims

1. A blood sugar constitution determination device comprising:
a storage unit that stores one or more pieces of question information that are used to judge a blood sugar constitution type and include a question regarding lifestyle;
an output unit that outputs the one or more pieces of question information;
an acceptance unit that accepts, from a user, answer information corresponding to the one or more pieces of question information;
a processing unit that determines a blood sugar constitution type of the user, using the one or more pieces of answer information accepted by the acceptance unit; and
an information output unit that outputs output information regarding the blood sugar constitution type.

2. The blood sugar constitution determination device according to claim 1,
wherein the storage unit includes:
a first question storage unit that stores one or more first questions that are used to judge presence or absence of a risk related to blood sugar and include a question regarding lifestyle; and
a second question storage unit that stores one or more second questions that are used to judge a blood sugar constitution type and include a question regarding lifestyle,
the one or more pieces of question information are the first questions or the second questions,
the acceptance unit includes:
a first answer acceptance unit that accepts first answers to the one or more first questions from the user; and
a second answer acceptance unit that accepts second answers to the one or more second questions from the user,
the processing unit includes:
a first judgment unit that judges presence or absence of a risk related to a blood sugar of the user, using the one or more first answers accepted by the first answer acceptance unit; and
a second judgment unit that determines a blood sugar constitution type of the user, using the one or more second answers accepted by the second answers acceptance unit, and
the output unit includes:
a first question output unit that outputs the one or more first questions; and
a second question output unit that outputs the one or more second questions when the first judgment unit judges that a risk is present.

3. The blood sugar constitution determination device according to claim 2,
wherein the second judgment unit additionally uses the one or more first answers accepted by the first answer acceptance unit to determine the blood sugar constitution type of the user.

4. The blood sugar constitution determination device according to claim 1, further comprising:
a learner storage unit that stores a learner acquired by performing machine learning processing on two or more pieces of training data that each contain one or more pieces of answer information and a blood sugar constitution type,
wherein the processing unit acquires a blood sugar constitution type by performing machine learning prediction processing, using the one or more pieces of answer information accepted by the acceptance unit and the learner.

5. The blood sugar constitution determination device according to claim 2, further comprising:
a learner storage unit that stores a learner acquired by performing machine learning processing on two or more pieces of training data that each contain one or more second answers and a blood sugar constitution type,
wherein the second judgment unit acquires a blood sugar constitution type by performing machine learning prediction processing, using the one or more second answers accepted by the second acceptance unit and the learner.

6. The blood sugar constitution determination device according to claim 2, further comprising:
an advice storage unit that stores one or more pieces of advice information in association with one or more type identifiers identifying the blood sugar constitution type,
wherein the information output unit outputs output information that contains one or more pieces of advice information associated with a type identifier identifying the type determined by the second judgment unit.

7. The blood sugar constitution determination device according to claim 6, further comprising:
an output information formation unit that acquires one or more pieces of advice information associated with a type identifier identifying the type determined by the second judgment unit, and forms output information that contains the type identifier and the one or more pieces of advice information,
wherein the information output unit outputs the output information formed by the output information formation unit.

8. The blood sugar constitution determination device according to claim 1,
wherein the one or more pieces of question information in the storage unit are questions acquired by a simple questionnaire acquisition device, and
the simple questionnaire acquisition device includes:
an estimated type acquisition unit that acquires an estimated type identifier, using answers to M questions out of answers that are answers to N questions and that correspond to any actual type identifier of two or more actual type identifiers, where M is less than N;
a question determination unit that, when an accuracy regarding a degree of matching between the estimated type identifier acquired by the estimated type acquisition unit and the actual type identifier satisfies a predetermined accuracy condition, determines M questions corresponding to the estimated type identifier; and
a simple questionnaire output unit that outputs a simple questionnaire that is information regarding a question group that is a group of the M questions determined by the question determination unit.

9. The blood sugar constitution determination device according to claim 2 or 3,
wherein the one or more first questions in the first question storage unit are questions acquired by a simple questionnaire acquisition device, and
the simple questionnaire acquisition device includes:
an estimated type acquisition unit that acquires estimated risk presence/absence information, using answers to M questions out of answers that are answers to N questions and that correspond to actual risk presence/absence information specifying presence or absence of the risk, where M is less than N;
a question determination unit that, when an accuracy regarding a degree of matching between the estimated risk presence/absence information acquired by the estimated type acquisition unit and the actual risk presence/absence information satisfies a predetermined accuracy condition, determines M questions corresponding to the estimated risk presence/absence information; and
a simple questionnaire output unit that outputs a simple questionnaire that is information regarding a question group that is a group of the M questions determined by the question determination unit.

10. The blood sugar constitution determination device according to claim 2 or 3,
wherein one or more kinds of questions of the one or more second questions in the second question storage unit are questions acquired by a simple questionnaire acquisition device, and
the simple questionnaire acquisition device includes:
an estimated type acquisition unit that acquires an estimated type identifier, using answers to M questions out of answers that are answers to N questions and that correspond to any actual type identifier of two or more actual type identifiers, where M is less than N;
a question determination unit that, when an accuracy regarding a degree of matching between the estimated type identifier acquired by the estimated type acquisition unit and the actual type identifier satisfies a predetermined accuracy condition, determines M questions corresponding to the estimated type identifier; and
a simple questionnaire output unit that outputs a simple questionnaire that is information regarding a question group that is a group of the M questions determined by the question determination unit.

11. The blood sugar constitution determination device according to any one of claims 1 to 10,
wherein the blood sugar constitution type is a combination of a type of insulin sensitivity and a type of insulin secretion.

12. A blood sugar constitution determination method that is realized using a storage unit that stores one or more pieces of question information that are used to judge a blood sugar constitution type and include a question regarding lifestyle, an output unit, an acceptance unit, a processing unit, and an information output unit, the blood sugar constitution determination method comprising:
an output step in which the output unit outputs the one or more pieces of question information;
an acceptance step in which the acceptance unit accepts, from a user, answer information corresponding to the one or more pieces of question information;
a processing step in which the processing unit determines a blood sugar constitution type of the user, using the one or more pieces of answer information accepted by the acceptance unit; and
an information output step in which the information output unit outputs output information regarding the blood sugar constitution type.

13. A recording medium having recorded thereon a program that enables a computer that can access a storage unit that stores one or more pieces of question information that are used to judge a blood sugar constitution type and include a question regarding lifestyle, to function as:
an output unit that outputs the one or more pieces of question information;
an acceptance unit that accepts, from a user, answer information corresponding to the one or more pieces of question information;
a processing unit that determines a blood sugar constitution type of the user, using the one or more pieces of answer information accepted by the acceptance unit; and
an information output unit that outputs output information regarding the blood sugar constitution type.
